(19) Europäisches Patentamt
European Patent Office
Office européen des brevets

(11) Veröffentlichungsnummer: **0 122 488 B1**

(12) # EUROPÄISCHE PATENTSCHRIFT

(45) Veröffentlichungstag der Patentschrift: **07.06.89**

(51) Int. Cl.[4]: **C 07 D 403/06**, A 61 K 31/495

(21) Anmeldenummer: **84102979.6**

(22) Anmeldetag: **17.03.84**

(54) **Bis-(Piperazinyl- bzw. Homopiperazinyl)-Alkane.**

(30) Priorität: **21.03.83 US 477008**

(43) Veröffentlichungstag der Anmeldung: **24.10.84 Patentblatt 84/43**

(45) Bekanntmachung des Hinweises auf die Patenterteilung: **07.06.89 Patentblatt 89/23**

(84) Benannte Vertragsstaaten: **AT BE CH DE FR GB IT LI LU NL SE**

(56) Entgegenhaltungen:
**CA–A– 675 224**
**Patent Abstracts of Japan Band 2, Nr. 34, 8. März 1978 Seite 4386C77 & JP-A-52-131590**
**Chemical Abstracts Band 54, Nr. 1, 10. Januar 1960, Columbus, Ohio, USA C.B. POLLARD et al. "Derivatives of piperazine. XXXIV. Some reactions of trimethylene chlorobromide with 1-arylpiperazine", Spalte 551c-i**
**Chemical Abstracts Band 63, Nr. 7, 27. September 1965, Columbus, Ohio, USA S. CHIAVARELLI et al. "Curare-like active quaternary ammonium derivatives of 1-benzylpiperazine", Spalten 8362f-8363a**
**Die Akte enthält technische Angaben, die nach dem Eingang der Anmeldung eingereicht wurden und die nicht in dieser Patentschrift enthalten sind.**

(73) Patentinhaber: **Boehringer Ingelheim Ltd.**
**90 East Ridge P.O. Box 368**
**Ridgefield, Conn 06877 (US)**

(72) Erfinder: **Devlin, John P., Dr.**
**57 Montgomery Street**
**Poughkeepsie New York 12601 (US)**
Erfinder: **McNeil, Daniel W.**
**21 Rita Drive RR 5**
**Box 407, New Fairfield Connecticut 06810 (US)**
Erfinder: **Keirns, James J., Dr.**
**55 Mill Plain Road Unit 17-2**
**Danbury Connecticut 06810 (US)**
Erfinder: **Barsumian, Edward L.**
**55 Mill Plain Road Unit 17-1**
**Danbury Connecticut 06810 (US)**

(74) Vertreter: **Milnes, Rodger et al**
**Boehringer Ingelheim Zentrale GmbH ZA Patente**
**Postfach 200**
**D-6507 Ingelheim am Rhein (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 122 488 B1

## Beschreibung

Die vorliegende Erfindung betrifft neue Bis-(Piperazinyl-bzw. Homopiperazinyl)-Alkane und deren physiologisch verträgliche Säureadditionssalze, Verfahren zur Herstellung der Verbindungen, diese enthaltende pharmazeutische Zusammensetzungen. Die beanspruchten Verbindungen können als Antiallergika und entzündungshemmende Mittel verwendet werden.

Stand der Technik

Verbindungen der Formel

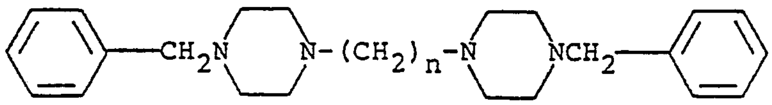

worin n eine der Zahlen 2, 6, 8, 9 oder 10 bedeutet, werden von S. Chiavarelli, P. Mazzeo, F. Costa und A. M. Russo in Farmaco, Ed. Sci. 20, 229 (1965) beschrieben ; sie besitzen eine curare-ähnliche Wirkung.

J. van Alpen beschreibt in Rec. Trav. Chim. 55, 835 (1936) die Synthese von Polyaminen der Formeln

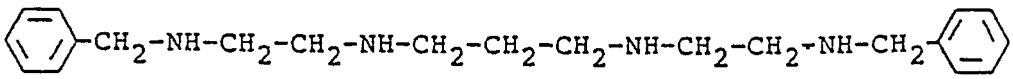

und

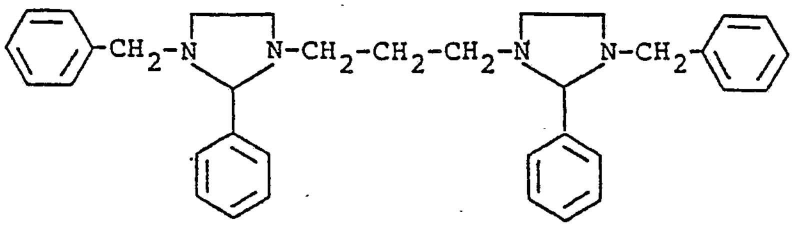

ohne Hinweis auf eine biologische Wirksamkeit.

Die Arbeit von C. B. Pollard, W. M. Lauter und N. O. Nuessle in J. Org. Chem. 24, 764 (1959) befasst sich mit der Herstellung von Verbindungen der Formel

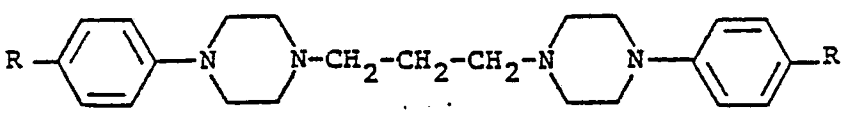

worin R Wasserstoff, Alkyl oder Halogen bedeutet. Auch hier werden keine Angaben zur Wirksamkeit gemacht.

Im Belgischen Patent Nr. 633,453 werden Verbindungen der Formel

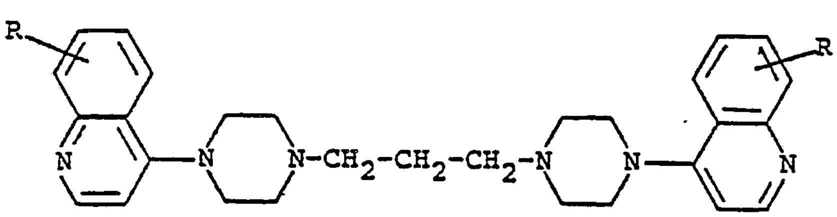

worin R Halogen oder Alkoxy bedeutet, mit Antimalaria-, anthelmintischer und amöbicider Wirkung beansprucht.

Schließlich beschreiben M. J. Dorokhova, V. A. Chernow, S. M. Minakova, O. Y. Tikhonova und A. N. Zamskaya, Khim.-Farm. Zh., 10, 36 (1976), (C. A. 85, 78079) Verbindungen der Formel

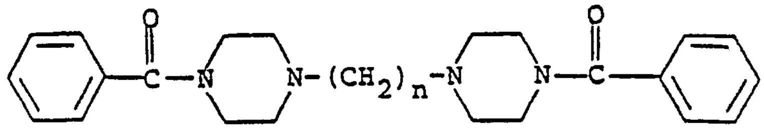

worin n eine der Zahlen 2, 3, 6 oder 10 bedeutet. Diese Verbindungen dienen als Ausgangsstoffe für die Verbindungen der allgemeinen Formel VIII vorliegenden Erfindung.

Patents Abs. of Japan 2 Nr. 34 (1978), 4386C77 offenbart N-substituierte Trimethoxybenzylpiperazine, die Herzmittel darstellen. C.A. 54, 1 (1960) 551c-i offenbart verschiedene Arylpiperazine jedoch ohne Angabe ihrer spezifischen pharmakologischen Wirksamkeit. C.A. 63, 7 (1965), 8362g-8363a offenbart 1-Benzylpiperidinderivate mit curare-ähnlicher Wirkung. CA-A-675 224 offenbart 1,4-Dipiperazino-butan-2.3-diole ; hierauf nimmt der Disclaimer in Anspruch 1 bezug. CA-A-675 224 offenbart lediglich, daß diese Verbindungen pharmazeutische Zwischenprodukte sind, nicht jedoch, daß sie selbst pharmakologisch wirksam sind.

Die vorliegende Erfindung betrifft neue Bis-(Piperazinyl-bzw. Homopiperazinyl)-Alkane der allgemeinen Formel (I)

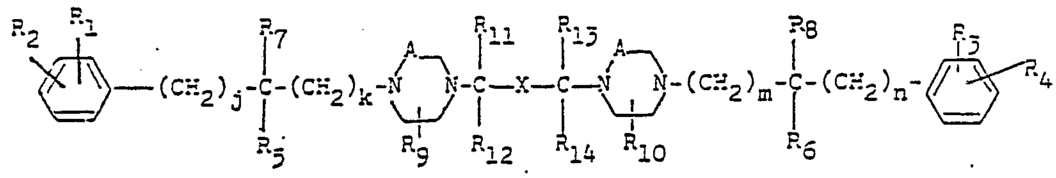 (I)

worin

$R_1$, $R_2$, $R_3$ und $R_4$, die gleich oder verschieden sein können, Wasserstoff, eine Alkylgruppe mit 1 bis 4 Kohlenstoffatomen, die Hydroxygruppe, eine Alkoxygruppe mit 1 bis 4 Kohlenstoffatomen, eine Acyloxygruppe mit 1 bis 4 Kohlenstoffatomen, Halogen, Trihalogenmethyl, Di-$C_{1-4}$-alkylamino, $C_{1-4}$-Alkoxycarbonyl, Nitro, Cyano oder Acyl mit 1 bis 3 Kohlenstoffatomen ;

$R_5$ und $R_6$, die gleich oder verschieden sein können, Wasserstoff, Methyl, Hydroxy, Carboxy, $C_{1-4}$-Alkoxycarbonyl, Hydroxymethyl, Phenyl oder p-Chlorphenyl ;

$R_7$ und $R_8$ Wasserstoff oder Methyl ;

j und k Indices von 0 bis 3, insgesamt jedoch nicht mehr als 4 ;

m und n Indices von 0 bis 3, insgesamt jedoch nicht mehr als 4 ;

A $-CH_2-$ oder $-CH_2-CH_2-$ :

oder

$R_5$ und $R_7$ zusammen oder $R_6$ und $R_8$ zusammen Oxo mit der Maßgabe, daß k oder m von 0 verschieden sind ; oder

$R_5$ und $R_7$ zusammen und $R_8$ und $R_{10}$ zusammen Oxo mit der Maßgabe, daß k und m von 0 verschieden sind ;

$R_9$ und $R_{10}$, die gleich oder verschieden sein können, Wasserstoff oder einen bis vier Methylsubstituenten an den Kohlenstoffatomen eines Piperazinrings ($A = -CH_2-$) ;

$R_{11}$, $R_{12}$, $R_{13}$ und $R_{14}$, die gleich oder verschieden sein können, Wasserstoff oder Methyl ; oder

$R_{11}$ und $R_{12}$ zusammen und/oder $R_{13}$ und $R_{14}$ zusammen Oxo ; und

X eine Alkylenkette mit 1 bis 2 Kohlenstoffatomen, die gegebenenfalls durch Hydroxy substituiert ist, bedeutet mit der Maßgabe daß wenn $A = -CH_2-$, $R_1$-$R_{14}$ Wasserstoff und j, k, m und n gleich 0 darstellen, X nicht 1,2 ethylendiol bedeuten kann, und deren physiologisch unbedenkliche Säureadditionssalze.

Eine bevorzugte Untergruppe bilden Verbindungen der allgemeinen Formel (II)

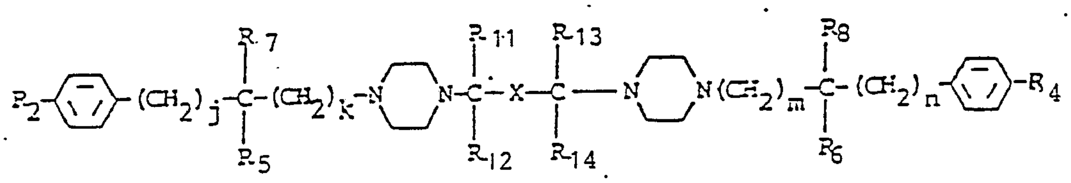 (II)

worin

j, k, m und n jeweils 0, 1 oder 2 ;

$R_2$ und $R_4$ Wasserstoff, Chlor, Methyl oder $C_{1-4}$-Alkoxy ;

$R_5$, $R_6$, $R_7$, $R_8$ sowie $R_{11}$, $R_{12}$, $R_{13}$, $R_{14}$ und X die oben angeführte Bedeutungen haben können

und deren physiologisch verträgliche Säureadditionssalze

Besonders bevorzugt sind Verbindungen der allgemeinen Formel (III)

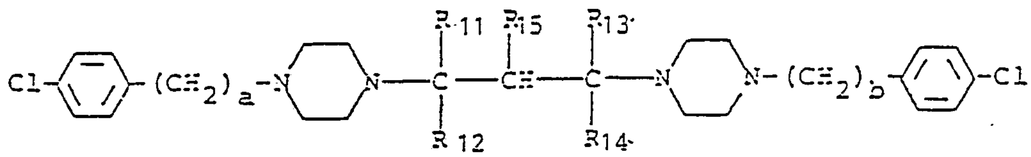

(III)

worin

$R_{11}$ und $R_{12}$ Wasserstoff oder zusammen Oxo ;
$R_{15}$ Wasserstoff oder Hydroxy ;
$R_{13}$ und $R_{14}$ Wasserstoff oder zusammen Oxo ; und
a und b jeweils 1, 2, 3 oder 4 bedeuten

und deren pharmazeutisch verträgliche Säureadditionssalze.

Die neuen Verbindungen können nach verschiedenen Verfahren erhalten werden :

Verfahren A

Umsetzung eines Equivalents einer Verbindung der allgemeinen Formel (IV)

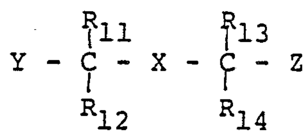

(IV)

worin $R_{11}$, $R_{12}$, $R_{13}$, $R_{14}$ und X die oben angegebene Bedeutung haben und Y und Z reaktive Gruppen bedeuten, die mit einem Amin unter Bildung einer Kohlenstoff- Stickstoffbindung reagieren, wie beispielsweise Chlor, Brom, Jod, aktivierter Ester, Hydroxy, Schwefelsäureester, und Sulfonsäureester mit einer Verbindung der allgemeinen Formel (V)

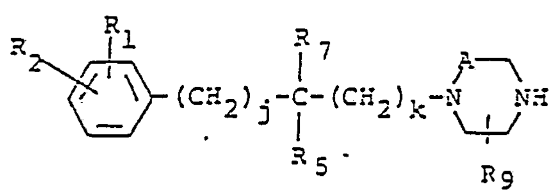

(V)

worin $R_1$, $R_2$, $R_5$, $R_7$, $R_9$, j, k und A die obengenannte Bedeutung besitzen.

Nach diesen Verfahren werden ausschließlich symmetrische Verbindungen der Formel (I) erhalten.

Verfahren B

Umsetzung einer Verbindung der allgemeinen Formel (VI)

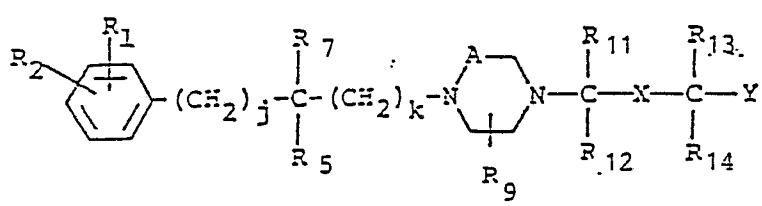

(VI)

worin $R_1$, $R_2$, $R_5$, $R_7$, $R_9$, $R_{11}$, $R_{12}$, $R_{13}$, $R_{14}$, X, j, k und A die oben angegebene Bedeutung haben, und Y eine reaktive Gruppe darstellt mit einer Verbindung der allgemeinen Formel (VII)

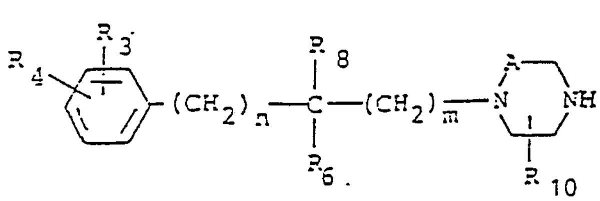

(VII)

worin $R_3$, $R_4$, $R_6$, $R_8$, $R_{10}$, m, n und A die oben angegebene Bedeutung haben.

Nach diesem Verfahren können sowohl symmetrische als auch unsymmetrische Verbindungen der allgemeinen Formel (I) erhalten werden.

Verfahren C

Verbindungen der allgemeinen Formel (I), die mit Bezug auf die zentrale Gruppe X symmetrisch sind, können hergestellt werden durch Reaktion einer Verbindung der allgemeinen Formel (VIII)

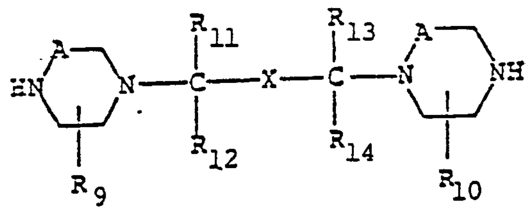

(VIII)

worin $R_9$, $R_{10}$, $R_{11}$, $R_{12}$, $R_{13}$, $R_{14}$, X und A die oben angegebene Bedeutung haben, mit einer Verbindung der allgemeinen Formel (IX)

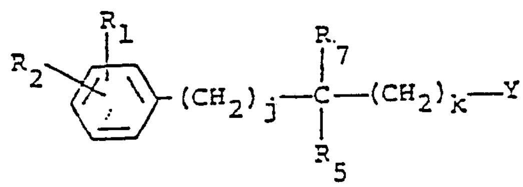

(IX)

worin $R_1$, $R_2$, $R_5$, $R_7$, Y, j und k die oben angegebene Bedeutung haben.

Verfahren D

Solche Verbindungen der allgemeinen Formel (I), in denen X ein Carbinol bedeutet, können auch hergestellt werden durch Umsetzung einer Verbindung der allgemeinen Formel (X)

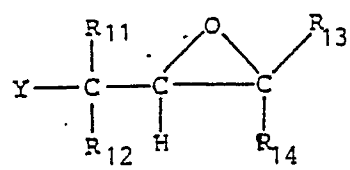

(X)

worin

$R_{11}$, $R_{12}$, $R_{13}$ und $R_{14}$, die oben angegebene Bedeutung haben,

Y die gleiche Bedeutung hat wie in Formel (IV),

mit einer Verbindung der allgemeinen Formel (VII).

Die in den Verfahren A bis D beschriebenen Kondensationsreaktionen können in Anwesenheit oder Abwesenheit eines Lösungsmittels durchgeführt werden. Verwendung finden können wäßrige oder organische interte Lösungsmittel, wobei die Wahl des Lösungsmittels von der Natur der Reaktionskomponenten abhängt. Beispiele solcher Lösungsmittel sind: Dimethylsulfoxid, Dimethylformamid, Dioxan, Ethoxyethanol und Alkanole mit bis zu 5 Kohlenstoffatomen, mit oder ohne Zusatz von Wasser; auch aromatische Kohlenwasserstoffe können verwendet werden. Vorzugsweise wird die Reaktion in Gegenwart eines säurebindenden Mittels wie Triethylamin, einem Alkalimetallcarbonat oder einem Alkalimetallhydroxid durchgeführt.

Die Reaktionstemperatur ist abhängig von den Ausgangsverbindungen und vom für diese Reaktion benützten Lösungsmittel und liegt zwischen Zimmertemperatur und der Rückflußtemperatur des Reaktionsgemischs. Die Reaktionszeit ist temperaturabhängig und reicht von einigen Minuten bis zu mehreren Stunden.

Ein Endprodukt der allgemeinen Formel I, in dem $R_5$ und/oder $R_6$ Hydroxy bedeuten, kann erhalten werden durch Hydrierung einer Verbindung, in der $R_5$ und $R_7$ und/oder $R_6$ und $R_8$ zusammen Oxo bedeuten mit üblichen Hydrierungsmitteln wie Natriumborhydrid in an sich bekannter Weise.

Man erhält so die entsprechende Hydroxyverbindung.

Die Verbindungen der allgemeinen Formel (I) sind basisch und bilden daher Additionssalze mit anorganischen oder organischen Säuren. Beispiele von nicht-toxischen, physiologisch verträglichen Säureadditionssalzen sind solche die man durch Umsetzung mit einer Halogenwasserstoffsäure, vorzugsweise Chlorwasserstoffsäure oder Bromwasserstoffsäure, mit Salpetersäure, Schwefelsäure, o-Phosphorsäure, Zitronensäure, Maleinsäure, Fumarsäure, Propionsäure, Buttersäure, Essigsäure, Bernsteinsäure, Methansulfonsäure, oder Benzolsulfonsäure p-Toluolsulfonsäure erhält.

Die Ausgangsverbindungen für die Verfahren A bis D sind bekannte Verbindungen oder können nach an sich bekannten Methoden hergestellt werden.

Zum Beispiel sind Verbindungen der allgemeinen Formel (V) beschrieben im Britischen Patent Nr. 480,358 und in J. Am. Chem. Soc., 66, 263 (1944).

Die Synthese von Verbindungen der allgemeinen Formel (VI) ist aus zahlreichen Puplikationen bekannt, zum Beispiel aus Helv. Chim. Acta 41, 1072 (1958) oder Monatshefte 87, 701 (1956).

Verbindungen der allgemeinen Formel (VIII) werden beschrieben in Britischen Patent Nr. 480,358 und Khim.-Farm. Zh. 10, 36 (1976), referiert in C.A. 85, 78079.

Die im folgenden aufgezählten Verbindungen der allgemeinen Formel (I) sowie ihre Säureadditionssalze können nach den oben beschriebenen Verfahren hergestellt werden.

1,3-Bis-[4-(hydroxybenzyl)-1-piperazinyl]-propan-tetrahydrochlorid,
1,3-Bis-[4-(4-chlorbenzyl)-1-piperazinyl]-propan-tetrahydrochlorid,
1,3-Bis-[4-(4-chlorbenzyl)-1-piperazinyl]-2-hydroxypropan,
1,4-Bis-[4-(4-chlorbenzyl)-1-piperazinyl]-butan-hemihydrat,
1,3-Bis-(4-benzyl-1-piperazinyl)-propan-tetrahydrochlorid,
1,3-Bis-[4-fluorbenzyl)-1-piperazinyl]-propan-tetrahydrochlorid,
1,3-Bis-[4-(4-chlorbenzyl)-1-piperazinyl]-1-oxopropantrihydrochlorid,
1,3-Bis-[4-(4-chlorbenzyl)-1-piperazinyl]-1-methylpropan-tetrahydrochlorid-hemihydrat,
1,3-Bis-[4-(4-chlorbenzhydryl)-1-piperazinyl]-propan-dihydrochlorid-dihydrat,
1-[4-(4-chlorbenzyl)-1-piperazinyl]-3-[4-(2-ethoxycarbonyl-2-phenylethyl)-1-piperazinyl]-propan-tetrahydrochlorid-monohydrat,
1-[4-(4-chlorbenzyl-1-piperazinyl]-3-(4-phenacyl-1-piperazinyl)-propan-tetrahydrochlorid-monohydrat,
1,3-Bis-(4-phenacyl-1-piperazinyl)-propan-tetrahydrochlorid-monohydrat,
1,3-Bis-[4-(2-phenyl-2-hydroxyethyl)-1-piperazinyl]-propan-tetrahydrochlorid,
1,3-Bis(4-phenethyl-1-piperazinyl)-propan-dihydrochlorid-dihydrat,
1-[4-(4-chlorbenzyl)-1-piperazinyl]-3-[4-(2-hydroxy-2-phenylethyl)-1-piperazinyl]-propan-tetrahydrochlorid,
1,3-Bis-[4-(4-chlorbenzyl)-1-piperazinyl]-1,3-dioxopropan-dihydrochlorid-monohydrat,
1,3-Bis-[4-(4-chlorphenethyl)-1-piperazinyl]-propan,
1,3-Bis-[4(1-phenylethyl)-1-piperazinyl]-propan-tetrahydrochlorid,
1,3-Bis-[4-(4-chlorbenzyl-2,5-dimethyl-1-piperazinyl]-propan-tetrahydrochlorid-dihydrat,
1,3-Bis-[4(4-methoxybenzyl)-1-piperazinyl]-propan,
1,3-Bis-[4-(3,4-dichlorbenzyl-1-piperazinyl]-propan-tetrahydrochlorid,
1,3-Bis-[4-(2-chlorbenzyl)-1-piperazinyl]-propan-tetrahydrochlorid,
1,3-Bis-[4-(4-methylbenzyl)-1-piperazinyl]-propan-tetrahydrochlorid,
1,3-Bis-[4-(3-chlorbenzyl)-1-piperazinyl]-propan-dihydrochlorid-monohydrat,
1,3-Bis-[4-(3-{4-chlorphenyl}-propyl)-1-piperazinyl]-propan-tetrahydrochlorid-monohydrat,
1,3-Bis-[4-(4-butoxybenzyl)-1-piperazinyl-propan,
1,3-Bis-[4-(4-acetoxybenzyl)-1-piperazinyl]-propan-tetrahydrochlorid,
1,3-Bis-[4-(4-brombenzyl)-1-piperazinyl]-propan,
1,3-Bis-[4-(4-chlor-3-trifluormethylbenzyl)-1-piperazinyl]-propan-tetrahydrochlorid
1,3-Bis-[4{4-(4-chlorbenzyl]-2,3,5,6-tetramethyl}-1-piperazinyl]-propan,
1,3-Bis-[4-{4-(4-chlorphenylbutyl)}-1-piperazinyl]-propan,
1-[4-(4-chlorbenzyl)-1-piperazinyl]-3-[4-(2-carboxy-2-phenylethyl)-1-piperazinyl]-propan-tetrahydrochlorid,
1,3-Bis-[4-(4-chlorbenzyl)-1-homopiperazinyl]-propan-tetrahydrochlorid,
1,3-Bis-[4-(3-{4-chlorphenyl}-propyl)-1-homopiperazinyl]-propan-tetrahydrochlorid,
1,3-Bis-[4-(4-chlorbenzyl)-1-homopiperazinyl]-1,3-dioxo-propan.

Die folgenden Beispiele dienen zur näheren Erläuterung der Erfindung :

## Beispiel 1

1,3-Bis-[4-(4-chlorbenzyl)-1-piperazinyl]-propan-tetrahydrochlorid

Eine Mischung von 10,5 g 1-(4-Chlorbenzyl)-piperazin, 9,5 g 1-Brom-3-chlorpropan und 100 ml Ethanol werden 17 Stunden unter Rückfluß erhitzt. Sodann wird das Lösungsmittel durch Rotationsverdampfung entfernt und das verbleibende Öl mit 200 ml 1M dibasischem Kaliumphosphat gemischt. Festes tribasisches Natriumphosphat wird solange zugegeben, bis ein pH-Wert von über 9 erreicht ist. Diese Mischung wird 5 mal mit 50 ml Ether extrahiert, der Ether verdampft, der Rückstand mit 100 ml 2M Phosphorsäure angesäuert und filtriert. Das wässrige Filtrat wird mit 2N Natronlauge basisch gestellt, wieder mit 250 ml Ether extrahiert und über Magnesiumsulfat getrocknet. Nach Einleiten vom gasförmigen Chlorwasserstoff und Umkristallisieren aus Ethanol/Wasser erhält man 5,8 g (39 % in der Theorie) der Titelverbindung in Form weißer Kristalle vom Fp. 261-274 °C (Zers.)

Beispiel 2

1,3-Bis-[4-(4-chlorbenzyl)-1-piperazinyl]-2-hydroxypropan

Ein Gemisch aus 4,4 g Epichlorhydrin, 20,1 g 1-(4-Chlorbenzyl)-piperazin, 6,0 g Triethylamin und 50 g Ethanol werden drei Tage unter Rückfluß erhitzt. Das Lösungsmittel wird durch Rotationsverdampfung aus dem Reaktionsgemisch entfernt und der Rückstand mit 2N Natronlauge basisch gestellt und 5 mal mit je 100 ml Ether extrahiert. Nach Trocknen des Etherextrakts über Magnesiumsulfat und Verdampfen des Lösungsmittels erhält man ein Öl, das sich beim Stehenlassen verfestigt. Nach Umkristallisieren aus Heptan erhält man 18,6 g (82 % der Theorie) 1,3-Bis-[4-(4-chlorbenzyl)-1-piperazinyl]-2-hydroxypropan in Form farblose Kristalle vom Fp. 85-86,5 °C.

Beispiel 3

1,4-Bis-[4-(4-chlorbenzyl)-1-piperazinyl]-butan-hemihydrat

Eine Mischung von 2,2 g 1,4-Dibrombutan, 4,2 g 1-(4-Chlorbenzyl-piperazin, 2,8 g wasserfreiem Kaliumcarbonat und 20 ml Ethanol werden 18 Stunden unter Rückfluß erhitzt. Das Lösungsmittel wird sodann unter vermindertem Druck abgezogen und das zurückbleibende Öl 16 Stunden auf 160 °C erhitzt. Danach wird das Öl in 50 ml heißem Wasser gelöst und 3 mal mit je 80 ml Ether extrahiert. Der Etherextrakt wird eingeengt und das verbleibende Öl an eine Kieselgelsäule chromatographiert (Laufmittel Methylenchlorid Methanol/Ammoniumhydroxid 45 : 5 : 1). Die erhaltene braungefärbte feste Masse wird in Aceton gelöst und mit Wasser die Titelverbindung in Form weißer Kristalle vom Fp. 101-103 °C ausgefällt.

Beispiel 4

1,3-Bis-(4-benzyl-1-piperazinyl)-propan-tetrahydrochlorid

Eine Mischung von 7,0 g 1-Benzylpiperazin, 3,2 g 1-Brom-3-chlorpropan, 4,0 g Triethylamin und 100 ml Ethanol werden 2 1/2 Stunden unter Rückfluß erhitzt. Sodann wird das Reaktionsgemisch in 1 Liter Ether gegossen und das ausgefallene Triethylaminsalz abfiltriert. Das Filtrat wird eingedampft und das verbleibende gelbe Öl in 100 ml Heptan gelöst und filtriert. Das Lösungsmittel wird durch Rotationsverdampfung entfernt und der Rückstand wiederum in 150 ml Ether gelöst. Durch Zufügen eines Überschusses an wasserfreier Salzsäure erhält man 8,8 g (82 % der Theorie) der Titelverbindung in Form weißer Kristalle vom Fp. 250-265 °C.

Beispiel 5

1,3-Bis-[4-(4-fluorbenzyl)-1-piperazinyl]-propantetrahydrochlorid

a) Eine Lösung von 29 g p-Fluorbenzylchlorid in 50 g Ethanol wird tropfenweise zu einer gerührten Lösung von 34,4 g Piperazin in 150 g Ethanol zugefügt. Durch ein kaltes Wasserbad wird die Reaktionstemperatur auf 20 °C gehalten. Das Reaktionsgemisch wird weitere 1 1/2 Stunden gerührt und dann in 2 Liter Ether gegossen. Vom ausgefallenen Piperazin-Hydrochlorid wird abfiltriert und das Filtrat bis zu einem öligen Rückstand eingedampft, der an Kieselgel chromatographiert wird (Laufmittel: Methylenchlorid/Methanol/Ammoniumhydroxid 45 : 5 : 1). Nach Aufarbeitung der entsprechenden Fraktionen erhält man 21,7 g (56 % der Theorie) 1-(4-Fluorbenzyl)-piperazin in Form einer farblosen Flüssigkeit.

b) Eine Mischung aus 5,8 g 1-(4-Fluorbenzyl-piperazin, 3,2 g 1-Brom-3-chlorpropan, 4,0 g Triethylamin und 50 ml Ethanol wird 3 Stunden unter Rückfluß erhitzt und dann in 1 Liter Ether gegossen. Niederschlag wird abfiltriert und das Filtrat zu einem Öl eingeengt. Dieses Öl wird in 100 ml Ether gelöst und mit einem Überschuß an wasserfreier Salzsäure das Tetrahydrochlorid ausgefällt, das nach Umkristallisieren aus Ethanol/Wasser in einer Ausbeute von 3,3 g (29 % der Theorie) in Form weißer Kristalle vom Fp. 228-237 °C (Zers.) erhalten wird.

Beispiel 6

1,3-Bis-[4-(4-chlor-benzyl)-1-piperazinyl]-1-oxo-propantrihydrochlorid

Eine Mischung von 2,0 g 1-(4-Chlorbenzyl)-piperazin, 1,0 g Triethylamin, 20 g Xylol und 0,8 g 3-Brompropionylchlorid werden 18 Stunden unter Rückfluß erhitzt und das Reaktionsgemisch wird filtriert

und das Filtrat mit einem Überschuß an mit Salzsäure gesättigtem Ether bis zur sauren Reaktion gegen Lackmus versetzt. Der erhaltene Niederschlag wird filtriert und aus Ethanol/Wasser umkristallisiert. Man erhält 1,2 g (45 % der Theorie) der Titelverbindung in Form weißer Kristalle vom Fp. 222-250 °C (Zers.).

Beispiel 7

1,3-Bis-[4-(4-chlorbenzyl)-1-piperazinyl]-1-methylpropantetrahydrochlorid-hemihydrat

Ein Gemisch von 7,3 g 1-(4-Chlorbenzyl)-piperazin, 2,8 g 1,3-Dibrombutan, 11,0 g Triethylamin und 50 ml Ethanol werden 48 Stunden unter Rückfluß erhitzt. Das Lösungsmittel wird durch Rotationsverdampfung entfernt, der Rückstand mit 100 ml Toluol vermischt und 24 Stunden unter Rückfluß erhitzt. Sodann wird das Gemisch in 1 Liter Ether gegossen und filtriert. Das Filtrat wird zu einem Öl eingeengt und dieses an Kieselgel chromatographiert (Laufmittel : Methylenchlorid/Methanol/Ammoniumhydroxid 45 : 5 : 1). Das erhaltene Öl wird in 100 ml Ether gelöst und mit einem Überschuß wasserfreier Salzsäure ausgefällt. Der Niederschlag wird in Wasser gelöst und durch Zufügen von Aceton erneut ausgefällt ; man erhält 1,2 g (11 % der Theorie) der Titelverbindung in Form weißer Kristalle vom Fp. 228-232 °C.

Beispiel 8

1,3-Bis-[4-(4-chlorbenzhydryl)-1-piperazinyl]-propandihydrochlorid-dihydrat

Eine Mischung aus 7,4 g N-(p-Chlorbenzhydryl)-piperazin, 2,0 g 1-Brom-3-chlorpropan, 1,6 g Triethylamin und 25 g Ethanol werden 15 Stunden unter Rückfluß erhitzt. Die Reaktionsmischung wird mit 5N Natronlauge alkalisch gestellt und 5 mal mit je 50 ml Methylenchlorid extrahiert. Der Extrakt wird über Magnesiumsulfat getrocknet und mit Chlorwasserstoff gesättigter Ether bis zur sauren Reaktion gegen Lackmus zugegeben. Der Niederschlag des Rohprodukts (2,5 = 2,7 % der Theorie) wird abfiltriert und durch Lösen in Methylenchlorid und anschließende Ausfällung durch Etherzugabe gereinigt. Man erhält weiße Kristalle der Titelverbindung vom Fp. 163-196 °C. (Zers.).

Beispiel 9

1-[4-(4-Chlorbenzyl)-1-piperazinyl]-3-[4-(2-ethoxycarbonyl-2-phenylethyl)-1-piperazinyl]-propan-tetrahydrochloridmonohydrat

a) Eine Mischung von 1,8 g Atropasäureethylester und 0,9 g Piperazin werden in einem Rundkolben gerührt. Nach dem Abklingen der exothermen Reaktion wird die Mischung auf 80 °C erhitzt und weitere 20 Minuten gerührt. Sodann wird das Reaktionsgemisch über Nacht bei Zimmertemperatur stehen gelassen. Die erhaltene feste Masse wird an Kieselgel chromatographiert, wobei man zunächst Ether und dann Methylenchlorid/Methanol/Ammoniumhydroxid im Verhältnis 45 : 5 : 1 als Laufmittel verwendet. Das 1-(2-Ethoxycarbonyl-2-phenylethyl)-piperazin (0,75 g = 29 % der Theorie) kommt mit dem zweiten Laufmittel von der Kolonne und wird für die nachfolgende Reaktion ohne weitere Reinigung eingesetzt.

b) Eine Mischung aus 3,0 g 1-Chlor-3-[4-(4-chlorbenzyl)-1-piperazinyl]-propan, 3,5 g Triethylamin, 3,7 g 1-(2-Ethoxycarbonyl-2-phenylethyl)piperazin und 50 ml Ethanol werden 2 Stunden unter Rückfluß erhitzt und anschließend in 1 Liter Ether gegossen. Die Mischung wird filtriert und das Filtrat eingedampft. Das verbleibende Öl wird durch Chromatographie an Kieselgel gereinigt (Laufmittel : Methylenchlorid/Methanol/Ammoniumhydroxid 45 : 5 : 1). Man erhält 3,0 g eines Öls, das in 150 ml Ether gelöst wird ; durch Zugabe eines Überschußes an wasserfreier Chlorwasserstoffsäure erhält man 3,4 g einer festen Masse. Diese wird wieder an Kieselgel chromatographiert, wobei als erstes Laufmittel Ether, als zweites ein Gemisch aus Methylenchlorid/Methanol/Ammoniumhydroxid im Verhältnis 45 : 5 : 1 verwendet wird. Das erhaltene Produkt wird, wie oben beschrieben, in das Hydrochlorid überführt, in Wasser gelöst und durch Zugabe von Aceton die Titelverbindung in einer Ausbeute von 1,1 g (15 % der Theorie) mit einem Fp. von 198-201 °C als weiße Kristalle ausgefällt.

Beispiel 10

1-[4-(4-Chlorbenzyl)-1-piperazinyl]-3-(4-phenacyl-1-piperazinyl)propan-tetrahydrochlorid-monohydrat

Eine Mischung von 4,1 g 1-Phenacylpiperazin, 5,7 g 1-Chlor-3-[4-(4-chlorbenzyl)-1-piperazinyl]propan, 2,6 g Triethylamin und 35 ml Ethanol werden 5 Stunden unter Rückfluß erhitzt. Das Lösungsmittel wird im Rotationsverdampfer entfernt, dem Rückstand 150 ml Wasser zugefügt, und das Gemisch 3 mal mit 150 ml Ether extrahiert. Die etherische Lösung wird eingedampft und das verbleibende Öl an Kieselgel mit den Laufmittel Methylenchlorid/Methanol/Ammoniumhydroxid (45 : 5 : 1) chromatographiert ; man erhält 7,0 g (50 % der Theorie) rohes 1-[4-(4-Chlorbenzyl)-1-piperazinyl]-3-(4-phenacyl-1-piperazinyl)propan in Form eines Öls. Dieses Öl wird in 200 ml Ether gelöst, mit einem Überschuß an wasserfreier Salzsäure das Hydrochlorid gefällt, die Fällung in Wasser aufgelöst und durch Zugabe von Aceton die Titelverbindung vom Fp. 211-218 °C ausgefällt.

Beispiel 11

1-[4-(4-Chlorbenzyl)-1-piperazinyl]-3-[4-(2-hydroxy-2-phenylethyl)-1-piperazinyl]propan-tetrahydrochlorid

Eine Lösung von 3,0 g 1-[4-(4-Chlorbenzyl)-1-piperazinyl]-3-(4-phenacyl-1-piperazinyl)propan in 50 ml Ethanol wird mit 3,0 g Natriumborhydrid gemischt. Die Mischung wird 4 Stunden gerührt und sodann das nicht umgesetzte Natriumborhydrid durch Zugabe von 25 ml Aceton zerstört. Die Lösungsmittel werden unter Vakuum entfernt und dem Rückstand 50 ml Wasser zugesetzt. Die Mischung wird 3 mal mit je 250 ml Ether extrahiert, das Lösungsmittel verdampft und das verbleibende Öl an Kieselgel mit dem Laufmittel Methylenchlorid/Methanol/Ammoniumhydroxid (45 : 5 : 1) chromatographiert. Die das Endprodukt enthaltende Fraktionen werden vereinigt, das Lösungsmittel verdampft, das verbleibende Öl in 100 ml Ether gelöst und das Hydrochlorid durch Zugabe von überschüssiger wasserfreier Salzsäure aufgefällt. Nach Lösen in Wasser und Ausfällen mit Aceton erhält man das Endprodukt in einer Ausbeute von 0,65 g (16 % der Theorie) in Form weißer Kristalle vom Fp. 240-248 °C. (Zers.)

Beispiel 12

1,3-Bis-(4-phenacyl-1-piperazinyl)propan-tetrahydrochlorid-monohydrat

Eine Mischung von 6,1 g 1-Phenacylpiperazin, 2,4 g 1-Brom-3-chlorpropan, 3,1 Triethylamin und 50 ml Ethanol werden 3 Stunden unter Rückfluß erhitzt. Das Lösungsmittel wird verdampft, dem Rückstand 250 ml Wasser zugefügt und das Gemisch 3 mal mit 150 ml Ether extrahiert. Nach Verdampfen des Ethers wird das verbleibende Öl an Kieselgel mit dem Laufmittel Methylenchlorid/Methanol/Ammoniumhydroxid (45 : 5 : 1) chromatographiert. Die Fraktionen mit hoher Reinheit werden vereinigt, das Lösungsmittel verdampft, das verbleibende Öl in 150 ml Ether gelöst und mit einem Überschuß wasserfreier Salzsäure ausgefällt. Nach Lösen in Wasser und Ausfällung durch Zugabe von Aceton erhält man 1,3 g (14 % Der Theorie) der Titelverbindung vom Fp. 194-204 °C in Form weißer Kristalle.

Beispiel 13

1,3-Bis-[4-(2-phenyl-2-hydroxyethyl)-1-piperazinyl]propantetrahydrochlorid

Eine Lösung von 2,5 g 1,3-Bis(4-phenacyl-1-piperazinyl) propan in 50 ml Ethanol wird mit 2,5 g Natriumborhydrid gemischt und 4 Stunden gerührt. Das überschüssige Natriumborhydrid wird durch Zugabe von 25 ml Aceton zerstört und die Lösungsmittel durch Rotationsverdampfung entfernt. Dem Rückstand werden 50 ml Wasser zugefügt und das Gemisch 3 mal mit je 150 ml Ether extrahiert. Nach Verdampfen des Ethers wird das verbleibende Produkt an Kieselgel mit dem Laufmittel Methylenchlorid/Methanol/Ammoniumhydroxid (45 : 5 : 1) chromatographiert. Das erhaltene Öl wird in 100 ml Ether gelöst und durch Zugabe von überschüssiger wasserfreier Chlorwasserstoffsäure die Titelverbindung in einer Ausbeute von 0,75 g (22 % der Theorie) vom Fp. 233-240 °C in Form weißer Kristalle ausgefällt.

Beispiel 14

1,3-Bis-(4-phenethyl-1-piperazinyl)-propan-dihydrochloriddihydrat

Eine Mischung aus 5,7 g 1-Phenethylpiperazin, 2,4 g 1-Brom-3-chlorpropan, 4,1 g Triethylamin und 30 ml Ethanol werden 3 Stunden unter Rückfluß erhitzt. Danach werden 50 ml Wasser zugefügt, die Mischung durch Rotationsverdampfung auf etwa 40 ml eingeengt und dieses Gemisch 3 mal mit je 150 ml Ether extrahiert. Die vereinigten Extrakte werden eingedampft, das verbleibende braune Öl in 150 ml Ether gelöst und durch Zugabe eines Überschußes wasserfreier Salzsäure das Hydrochlorid ausgefällt. Es wird in Wasser gelöst und durch Zugabe von Aceton 3,1 g (37 % der Theorie) der Titelverbindung vom Fp. 210-225 °C als weiße Kristalle erhalten.

Beispiel 15

1,3-Bis-[4-(4-chlorbenzyl)-1-piperazinyl]-1,3-dioxopropandihydrochlorid-monohydrat

Eine Mischung von 4,2 g 1-(4-Chlorbenzyl)piperazin, 1,4 g Malonsäure-dichlorid, 10 g Methylenchlorid und 2,0 g Triethylamin werden 60 Stunden gerührt. Das Reaktionsgemisch wird mit 2N Natronlauge alkalisch gestellt, die organische Schicht abgetrennt und die wässrige Phase 3 mal mit je 50 ml Ether, anschließend 3 mal mit je 50 ml Methylenchlorid extrahiert. Die organischen Phasen werden vereinigt und mit 100 ml 2N Chlorwasserstoffsäure gemischt. Die wässrige Phase wird abgetrennt und mit 2N Natronlauge basisch gestellt. Das verbleibende Öl wird gesammelt und an Kieselgel mit dem Laufmittel Methylenchlorid/Methanol/Ammoniumhydroxid (200 : 5 : 1) extrahiert. Die geeigneten Fraktionen werden

vereinigt, das Lösungsmittel verdampft, das verbleibende Öl in 100 ml Ether gelöst und mit einem Überschuß wasserfreier Salzsäure ausgefällt. Die Ausfällung wird aus Ethanol umkristallisiert; man erhält 1,3 g (22 % der Theorie) der Titelverbindung vom Fp. 199-206 °C in Form leicht gelblicher Kristalle.

Beispiel 16

1,3-Bis-[4-(4-chlorphenethyl)-1-piperazinyl]-propan

Eine Mischung von 6,7 g 1-(4-Chlorphenethyl)piperazin, 2,4 g 1-Brom-3-chlorpropan, 3,1 g Triethylamin und 20 ml Ethanol wird 3 Stunden unter Rückfluß erhitzt. Das Reaktionsgemisch wird mit 50 ml Wasser verdünnt und durch Rotationsverdampfung auf etwa 50 ml eingeengt. Das verbleibende Gemisch wird 3 mal mit je 150 ml Ether extrahiert und der Extrakt eingeengt. Die zurückbleibende farblose feste Masse wird aus Heptan umkristallisiert; man erhält 3,3 g (45 % der Theorie) der Titelverbindung vom Fp. 87-88 °C in Form weißer Kristalle.

Beispiel 17

1,3-Bis-[4-(1-phenylethyl)-1-piperazinyl]-propan-tetrahydrochlorid

Eine Mischung aus 7,6 g 1-(1-Phenylethyl)piperazin, 3,2 g 1-Brom-3-chlorpropan und 50 ml Ethanol wird 5 Stunden unter Rückfluß erhitzt. Sodann wird das Lösungsmittel unter Vakuum entfernt, 50 ml Wasser zugefügt und die Mischung 3 mal mit je 150 ml Ether extrahiert. Das ethärische Lösungsmittel wird verdampft und das verbleibende Öl an Kieselgel chromatographiert (Laufmittel Methylenchlorid/Methanol/Ammoniumhydroxid 45 : 5 : 1). Das so erhaltene gelbe Öl wird in 150 ml Ether gelöst und mit überschüssigem wasserfreiem Chlorwasserstoff 6,1 g (51 % der Theorie) der Titelverbindung ausgefällt; nach dem Umkristallisieren aus Ethanol/Wasser erhält man weiße Kristalle vom Fp. 236-246 °C (Zers.)

Beispiel 18

1,3-Bis[4-(4-chlorbenzyl)-2,5-dimethyl-1-piperazinyl]-propan-tetrahydrochlorid-dihydrat

a) Eine Lösung von 16 g p-Chlorbenzylchlorid in 75 ml Ethanol wird tropfenweise zu einer Lösung von 25 g 2,5-Dimethylpiperazin in 75 ml Ethanol zugegeben, die Mischung über Nacht gerührt und dann filtriert. Das Lösungsmittel wird durch Vakkumdestilation entfernt, der Rückstand 3 mal mit 350 ml Ether extrahiert, das Lösungsmittel verdampft und das verbleibende Öl an Kieselgel chromatographiert (Laufmittel Methylenchlorid/Methanol/Ammoniumhydroxid 45 : 5 : 1). Man erhält so 9,1 g (38 % der Theorie) 1-(4-Chlorbenzyl)-2,5-dimethylpiperazin als farblose Flüssigkeit; sie wird ohne weitere Reinigung für die folgende Umsetzung verwendet.

b) Ein Gemisch aus 6,0 g 1-(4-Chlorbenzyl)-2,5-dimethylpiperazin, 2,0 g 1-Brom-3-chlorpropan, 3,2 g Triethylamin und 50 ml Ethanol werden 6 Stunden unter Rückfluß erhitzt. Das Lösungsmittel wird im Vakuum entfernt, dem Rückstand 50 ml Wasser zugefügt und die Mischung 3 mal mit 150 ml Ether extrahiert. Das Lösungsmittel wird verdampft und das verbleibende Öl an Kieselgel chromatographiert (Laufmittel Methylenchlorid/Methanol Ammoniumhydroxid 45 : 5 : 1). Das erhaltene Produkt wird in 150 ml Ether gelöst und mit einem Überschuß wasserfreier Salzsäure ausgefällt. Der Niederschlag wird in Wasser gelöst und durch Zugabe von Aceton 0,7 g der Titelverbindung in Form weißer Kristalle vom Fp. 204-214 °C erhalten.

Beispiel 19

1,3-Bis-[4-(4-methoxybenzyl)-1-piperazinyl]-propan

Eine Mischung von 4,1 g 1-(p-Methoxybenzyl)piperazin, 1,6 g 1-Brom-3-chlorpropan, 25 ml Ethanol und 2,5 ml Triethylamin werden 5 Stunden unter Rückfluß erhitzt. Das Lösungsmittel wird im Vakuum entfernt, der Rückstand mit 25 ml Wasser vermischt und mit Ether extrahiert. Nach Verdampfen des Lösungsmittels wird ein gelbes Öl erhalten, das sich beim Stehen verfestigt. Nach zweimaligem Umkristallisieren aus Heptan erhält man 2,3 g (51 % der Theorie) der Titelverbindung in Form weißer Kristalle vom Fp. 86-87 °C.

Beispiel 20

1,3-Bis-[4-(3,4-dichlorbenzyl)-1-piperazinyl]-propantetrahydrochlorid

Eine Mischung von 5,2 g 1-(3,4-Dichlorbenzyl)-piperazin, 2,2 g 1-Brom-3-chlorpropan, 3,0 g Triethylamin und 20 g Ethanol werden unter Rückfluß erhitzt. Nach einer Stunde werden weitere 0,06 g 1-Brom-3-chlorpropan zugefügt, nach einer weiteren Stunden nochmals 0,06 g. Die Mischung wird über Nacht

unter Rückfluß erhitzt und anschließend im Vakuum das Lösungsmittel abgezogen; man erhält eine gummiartige feste Masse, die mit 150 ml Ether vermischt und filtriert wird. Das Filtrat wird über Magnesiumsulfat getrocknet und danach so lange mit Chlorwasserstoff gesättigter Ether zugegeben bis die Mischung auf Lackmus sauer reagiert. Der erhaltene Niederschlag wird in wenig Wasser gelöst und konzentrierte Salzsäure bis zur Bildung eines Niederschlags tropfenweise zugefügt. Die Zugabe der Salzsäure wird so lange fortgesetzt, bis sich kein weiterer Niederschlag bildet. Nach Filtrieren und Trocknen im Vakuum erhält man 4,5 g (44 % der Theorie) der Titelverbindung in Form weißer Nadeln vom Fp. 245-251 °C. (Zers.)

Beispiel 21

1,3-Bis[4-(2-chlorbenzyl)-1-piperazinyl]-propantetrahydrochlorid

Ein Gemisch aus 8,4 g 1-(2-Chlorbenzyl)piperazin, 3,2 g 1-Brom-3-chlorpropan, 4,0 g Triethylamin und 30 g Ethanol werden über Nacht unter Rückfluß erhitzt. Danach wird das Lösungsmittel im Vakuum abgezogen, der Rückstand mit 150 ml Ether vermischt und filtriert. Das Filtrat wird über Magnesiumsulfat getrocknet und danach mit Chlorwasserstoff gesättigtem Ether langsam bis zur sauren Reaktion auf Lackmus zugegeben. Der erhaltene Niederschlag wird filtriert, getrocknet und gewogen. Man erhält 6,1 g (50 % der Theorie) der Titelverbindung, die nach Umkristallisieren aus Ethanol/Wasser in Form weißer Kristalle vom Fp. 251-255 °C vorliegt.

Beispiel 22

1,3-Bis-[4-(4-methylbenzyl)-1-piperazinyl]-propantetrahydrochlorid

Eine Mischung aus 5,7 g 1-(4-Methylbenzyl)piperazin, 2,4 g 1-Brom-3-chlorpropan, 25 ml Ethanol und 3,0 g Triethylamin werden 5 Stunden unter Rückfluß erhitzt. Das Lösungsmittel wird im Vakuum entfernt und der Rückstand mit 40 ml Wasser vermischt. Die wässrige Mischung wird 3 mal mit 150 ml Ether extrahiert; nach Verdampfen des Ethers erhält man ein leicht gelbliches Öl das sich beim Stehen verfestigt. Dieses Produkt wird in 50 ml Ether gelöst und mit Chlorwasserstoff gesättigter Ether bis zur sauren Reaktion auf Lackmus zugefügt. Der erhaltene Niederschlag wird in 20 ml Wasser gelöst und durch Zugabe von Aceton die Titelverbindung in einer Ausbeute von 5,8 g (68 % der Theorie) in Form weißer Kristalle vom Fp. 245-252 °C (Zers.) ausgefällt.

Beispiel 23

1,3-Bis-[4-(3-chlorbenzyl)-1-piperazinyl]-propandihydrochlorid-monohydrat

Eine Mischung aus 6,3 g 1-(3-Chlorbenzyl)piperazin, 2,4 g 1-Brom-3-chlorpropan, 50 ml Ethanol und 3,0 g Triethylamin werden 4 Stunden unter Rückfluß erhitzt. Sodann werden 70 ml Wasser zugefügt, das Gemisch unter Vakuum auf etwa 70 ml eingeengt und die so erhaltene wässrige Mischung 3 mal mit je 150 ml Ether extrahiert. Nach dem Einengen des Extraktes in Vakuum erhält man ein rötlichgelbliches Öl, das an Silikagel chromatographiert wird (Laufmittel Methylenchlorid/Methanol/Ammoniumhydroxid 45 : 5 : 1). Das so erhaltene Öl wird in 50 ml Ethanol gelöst und mit Chlorwasserstoff gesättigter Ether bis zur sauren Reaktion auf Lackmus zugegeben. Der Niederschlag wird abfiltriert, getrocknet und aus Wasser umkristallisiert. Man erhält 5,6 g (68 % der Theorie) der Titelverbindung in Form weißer Kristalle vom Fp. 248-257 °C (Zers.).

Beispiel 24

1,3-Bis-[4-(3-{4-chlorphenyl}-propyl)-1-piperazinyl]-propan-tetrahydrochlorid-monohydrat

a) Ein Gemisch aus 40,6 g 3-(4-Chlorphenyl)propylchlorid, 130,0 g wasserfreiem Piperazin und 550 ml Ethanol werden 2 Stunden unter Rückfluß erhitzt. Das Lösungsmittel wird im Vakuum entfernt und der Rückstand an Kieselgel chromatographiert (Laufmittel Methylenchlorid/Methanol/Ammoniumhydroxid 45 : 5 : 1). Die entsprechenden Fraktionen werden vereinigt und zu einem Öl eingeengt, das mit 1 400 ml 1N Salzsäure vermischt und dann filtriert wird. Das Filtrat wird mit konzentrierter wässriger Natronlauge auf pH 10 gebracht und anschließend 4 mal mit je 200 ml Ether extrahiert. Der Extrakt wird über Magnesiumsulfat getrocknet und zu einem Öl eingeengt, das sich beim Stehen verfestigt. Das so erhaltene 1-[3-(4-Chlorphenyl)propyl]piperazin vom Fp. 54-62 °C wird ohne weitere Reinigung für den nächsten Schritt eingesetzt.

b) Eine Mischung aus 7,2 g 1-[3-(4-Chlorphenyl)propyl] piperazin, 2,4 g 1-Brom-3-chlorpropan, 3,5 g Triethylamin und 30 ml Ethanol werden 6 Stunden unter Rückfluß erhitzt. Sodann wird das Lösungsmittel im Vakuum abgezogen, der Rückstand mit 40 ml Wasser vermischt und 3 mal mit je 150 ml Ether extrahiert. Der Extrakt wird zu einem gelben Öl eingeengt, das an Kieselgel chromatographiert wird

(Laufmittel Methylenchlorid/Methanol/Ammoniumhydroxid (45 : 5 : 1). Man erhält so 4,6 g (59 % der Theorie) 1,3-Bis-[4-(3-{4-chlorphenyl}-propyl)-1-piperazinyl]-propan als farbloses Öl. 3,0 g dieses Öls werden in 100 ml Ether gelöst und bis zur sauren Reaktion auf Lackmus mit Chlorwasserstoff gesättigter Ether zugegeben. Der erhaltene Niederschlag wird in 25 ml Wasser gelöst und so lange Aceton zugegeben, bis die Ausfällung beendet ist. Man erhält 2,7 g (41 % der Theorie) der Titelverbindung vom Fp. 245-246 °C (Zers.) in Form weißer Kristalle.

Beispiel 25

1,3-Bis-[4-(4-chlor-3-trifluormethylbenzyl)-1-piperazinyl]-propan-tetrahydrochlorid

Eine Mischung aus 11,5 g 3-Chlor-4-trifluormethylbenzylchlorid, 5,3 g 1,3-Bis(1-piperazinyl)propan, 50 g Ethanol und 7,0 g Triethylamin werden 16 Stunden unter Rückfluß erhitzt. Danach wird das Reaktionsgemisch in Vakuum eingeengt, der Rückstand mit 150 ml Wasser vermischt und das Gemisch 5 mal mit je 100 ml Ether extrahiert. Die vereinigten Etherextrakte werden 3 mal mit je 100 ml 1M Natriumcarbonatlösung gewaschen, über Magnesiumsulfat getrocknet, filtriert und zu 6,8 g eines gelben Öls eingeengt. Dieses Öl wird in 100 ml Hexan gelöst und filtriert. Das Filtrat wird 3 mal mit je 20 ml 2 %iger wässriger Essigsäure extrahiert, wobei das gewünschte Produkt im 2. und 3. Extrakt erscheint (Prüfung durch Dünnschichtchromatographie). Diese Extrakte werden vereinigt, mit 2N Natronlauge stark alkalisch gestellt und mit 100 ml Ether/Hexan (1 : 1) extrahiert. Der Extrakt wird über wasserfreiem Kaliumcarbonat getrocknet und anschließend das Lösungsmittel verdampft. Das verbleibende Öl (2,6 g) wird in 10 ml Methanol gelöst ; nach Zufügen von 30 ml von mit Chlorwasserstoff gesättigtem Ether erhält man einen weißen Niederschlag. Weitere 50 ml Ether werden zugegeben und der Niederschlag abfiltriert und aus Methanol umkristallisiert. Man erhält die Titelverbindung in Form farbloser Kristalle vom Fp. 265-268 °C (Zers. > 250 °C).

Beispiel 26

1,3-Bis-[4-(4-hydroxybenzyl)-1-piperazinyl]-propan

Eine Mischung aus 4,5 g 1,3-Bis[4-(4-methoxybenzyl)-1-piperazinyl]propan (siehe Beispiel 19) und 250 ml 49 %iger Bromwasserstoffsäure werden 2 Stunden unter Rückfluß erhitzt, sodann abgekühlt und mit 125 ml Wasser verdünnt. Die wässrige Lösung wird filtriert und mit 2N Natronlauge auf pH 8 gebracht. Dieses Gemisch wird 3 mal mit jeweils 150 ml Ethanol extrahiert, der Alkohol durch Rotationsverdampfung entfernt und der Rückstand an Kieselgel chromatographiert (Laufmittel : Methylenchlorid mit 1 % Ammoniumhydroxid und 10 % Methanol). Man erhält 1,3 g (31 % der Theorie) der Titelverbindung vom Fp. 197-201 °C in Form weißer Kristalle.

Beispiel 27

1,3-Bis-[4-(4-brombenzyl)-1-piperazinyl]-propan-tetrahydrochlorid

Ein Gemisch aus 3,8 g 1-(4-Brombenzyl)piperazin, 7 g Ethanol, 1,2 g 1-Brom-3-chlorpropan und 1,6 g Triethylamin werden 18 Stunden unter Rückfluß erhitzt. Das Lösungsmittel wird durch Rotationsverdampfung entfernt, der Rückstand mit 50 ml Wasser gemischt und mit 2N Natronlauge auf pH 10 gebracht. Dieses Gemisch wird 2 mal mit Ether extrahiert (75 + 25 ml), die vereinigten Extrakte 2 mal mit je 25 ml Wasser gewaschen, über Magnesiumsulfat getrocknet und filtriert. Diese Lösung wird mit Chlorwasserstoff gesättigtem Ether bis zur sauren Reaktion versetzt und der ausfallende Niederschlag abfiltriert und mit 100 ml Ethanol vermischt. Das Gemisch wird auf Rückflußtemperatur gebracht und tropfenweise mit Wasser versetzt, bis es in Lösung gegangen ist. Beim Abkühlen kristallisiert das Endprodukt aus, das abfiltriert wird. Man erhält 4,2 g (81 % der Theorie) der Titelverbindung vom Fp. 237-243 °C in Form weißer Kristalle.

Beispiel 28

1,3-Bis-[4-(4-chlorbenzyl)-1-homopiperazinyl]-propantetrahydrochlorid

Ein Gemisch aus 4,5 g 1-(4-Chlorbenzyl)-homopiperazin, 1,6 g 1-Brom-3-chlorpropan und 20 g Ethanol werden 18 Stunden unter Rückfluß erhitzt. Das Lösungsmittel wird unter Vakuum entfernt und das verbleibende Gemisch 3 mal mit je 75 ml Ether extrahiert. Nach dem Verdampfen des Ethers verbleibt ein Öl, das an Silikagel chromatographiert wird (Laufmittel Methylenchlorid, enthaltend 0,5 % konzentriertes Ammoniumhydroxid und 2,5 % Methanol). Die das Endprodukt enthaltenden Fraktionen werden vereinigt, das Lösungsmittel verdampft, das verbleibende Öl in Ether gelöst und die Lösung filtriert. Die verbleibende Etherlösung wird mit Chlorwasserstoff gesättigtem Ether bis zur sauren Reaktion auf Lackmus versetzt. Der sich bildende Niederschlag wird getrocknet ; man erhält 1,4 g (22 % der Theorie)

der Titelverbindung in Form weißer Kristalle, die nach Umkristallisieren aus wässrigem Ethanol einem Fp. vom 218-224 °C aufweist (Zers.).

Beispiel 29

1,3-Bis-[4-(4-{4-chlorphenyl}-butyl)-1-piperazinyl]-propan-tetrahydrochlorid

a) Eine Mischung vom 26,4 g 4-(4-Chlorphenyl)butylchlorid, 86,1 g wasserfreiem Piperazin und 250 ml Ethanol werden über Nacht unter Rückfluß erhitzt. Das Lösungsmittel wird verdampft und der Rückstand 2 mal an Kieselgel chromatographiert (Laufmittel Methylenchlorid/Methanol/Ammoniumhydroxid 45 : 5 : 1). Die erste Fraktion enthält in der Hauptsache überschüssiges Piperazin, die zweite enthält 15,2 g 1-[4-(4-Chlorphenyl)-butyl]-piperazin als farbloses Öl, das sich beim Stehen verfestigt (Fp. 139-145 °C). Die das Rohprodukt wird ohne weitere Reinigung für den nächsten Schritt verwendet.

b) Eine Mischung aus 1,5 g 1-[4-(4-Chlorphenyl)butyl] piperazin, 0,5 g 1-Brom-3-chlorpropan, 0,7 g Triethylamin und 10 g Ethanol werden über Nacht unter Rückfluß erhitzt. Nach Zufügen von 3,5 ml 2N Natronlauge wird das Lösungsmittel im Vakuum entfernt. Der Rückstand wird mit Methylenchlorid extrahiert und der Extrakt an Kieselgel chromatographiert (Laufmittel Methylenchlorid/Methanol/Ammoniumhydroxid 90 : 10 : 1). Die das gewünschte Produkt enthaltenden Fraktionen werden zu einem Öl eingeengt und dieses in Ether gelöst. Nach Zugabe von mit Chlorwasserstoff gesättigtem Ether erhält man eine Fällung von 0,7 g (31 % der Theorie) der Titelverbindung als hellbraune feste Masse, die durch Umkristallisieren aus Ethanol/Masser in weiße Kristalle vom Fp. 213-217 °C (Zers.) umgewandet werden kann.

Beispiel 30

1,3-Bis-[4-(4-acetoxybenzyl)-1-piperazinyl]-propan

Eine Mischung aus 2,0 g 1,3-Bis-[4-(4-hydroxybenzyl)-1-piperazinyl] propan (siehe Beispiel 26), 1,0 g Pyridin und 50 g Essigsäureanhydrid werden über Nacht bei Zimmertemperatur gerührt. Das Gemisch wird unter Vakuum zu einem bernsteinfarbigen Öl eingeengt, das mit 100 ml Phosphat-Puffer (pH 8) gemischt und 3 mal mit je 100 ml Ether extrahiert wird. Der Extrakt wird über Magnesiumsulfat getrocknet und zu einer weißen festen Masse eingeengt. Nach Umkristallisieren aus Heptan erhält man 1,9 g (78 % der Theorie) der Titelverbindung in Form weißer Kristalle vom Fp. 102-105 °C.

Beispiel 31

1,3-Bis-[4-(4-butoxybenzyl)-1-piperazinyl]-propantetrahydrochlorid

Ein Gemisch aus 1,0 g 1,3-Bis[4-hydroxybenzyl)-1-piperazinyl] propan (siehe Beispiel 26), 10 ml 2N Natronlauge, 0,2 g Tetrabutylammoniumhydroxid (40 % in Wasser) und 5,0 g 1-Brombutan werden 3 Stunden auf dem Dampfbad erhitzt. Sodann wird das Gemisch 3 mal mit je 75 ml Ether extrahiert, über Magnesiumsulfat getrocknet und zu einem farblosen Öl eingeengt, das sich beim Stehen verfestigt. Dieses Produkt wird in 100 ml Ether gelöst und mit Chlorwasserstoff gesättigtem Ether bis zur Beendigung des Niederschlags versetzt. Nach dem Filtrieren erhält man 1,4 g (91 % der Theorie) der Titelverbindung, die nach dem Umkristallisieren aus Ethanol/Wasser in Form weißer Kristalle vom Fp. 207-218 °C vorliegt.

Beispiel 32

1,3-Bis-[4-(4-chlorbenzyl)-2,3,5,6-tetramethyl-1-piperazinyl]-propan-tetrahydrochlorid

Eine Mischung aus 3 g 1-(4-Chlorbenzyl)-2,3,5,6-tetramethylpiperazin, 1 g 1-Brom-3-chlorpropan und 1 g Triethylamin wird 24 Stunden in 20 ml Ethanol unter Rückfluß erhitzt. Danach wird das Lösungsmittel im Vakuum entfernt, das verbleibende Öl in Wasser gelöst und mit Ether extrahiert. Der Etherextrakt wird mit Wasser gewaschen und über Magnesiumsulfat getrocknet. Man erhält die Titelverbindung aus der etherischen Lösung durch Ausfällen mit einem Überschuß an etherischer Salzsäure und Umkristallisieren aus Ethanol in Form weißer Kristalle.

Beispiel 33

1-[4-(4-Chlorbenzyl)-1-piperazinyl]-3-[4-(2-carboxy-2-phenylethyl)-1-piperazinyl]-propan-tetrahydrochlorid

Eine Suspension von 340 mg 1-[4-(4-Chlorbenzyl)-1-piperazinyl]-3-[4-(2-ethoxycarbonyl-2-phenylethyl)-1-piperazinyl] propan-tetrahydrochlorid in 10 ml Ethanol werden mit 1 ml wässriger 5M Natronlauge versetzt und eine Stunde unter Rückfluß erhitzt. Sodann wird das Reaktionsgemisch unter

vermindertem Druck zur Trockne eingedampft. Der Rückstand wird mit 1N Salzsäure (3 ml) vermischt und diese Mischung 2 mal mit je 10 ml Ether extrahiert. Der Extrakt wird verworfen und der pH-Wert der wässrigen Phase mit 1N Salzsäure auf 5,5 eingestellt. Sodann werden 10 ml gesättigte Kochsalzlösung zugegeben und die Mischung 2 mal mit je 10 ml Butanol extrahiert. Der Butanolextrakt wird filtriert und mit einem Überschuß etherischer Salzsäure versetzt. Der weiße Niederschlag wird gesammelt und aus wässrigem Ethanol umkristallisiert. Man erhält 140 ml (40 % der Theorie) der Titelverbindung in Form weißer Kristalle vom Fp. 230-245 °C (Zers.).

Beispiel 34

1,3-Bis-[4-(3-{4-chlorphenyl}-propyl)-1-homopiperazinyl]-propan-tetrahydrochlorid

Ein Gemisch aus 3,8 g 1-[3-(4-Chlorphenyl)propyl]-homopiperazin, 1,2 g 1-Brom-3-chlorpropan, 1,8 g Triethylamin werden in 10 ml Ethanol 18 Stunden unter Rückfluß erhitzt. Das Reaktionsgemisch wird unter vermindertem Druck eingedampft, der Rückstand mit Wasser vermischt und mit Ether extrahiert. Der Ether wird verdampft und der Rückstand an Kieselgel chromatographiert (Laufmittel Methylenchlorid/Methanol/Ammoniumhydroxid 35 : 5 : 1). Das erhaltene Öl wird in Ether gelöst und die Titelverbindung mit einem Überschuß an etherischer Salzsäure ausgefällt. Nach Umkristallisieren aus Ethanol erhält man das 1,3-Bis[4-(3-{4-chlorphenyl}-propyl)-1-homopiperazinyl] propan tetrahydrochlorid in kristalliner Form.

Beispiel 35

1,3-Bis-[4-(4-chlorbenzyl)-1-homopiperazinyl]-1,3-dioxopropan-dihydrochlorid

Zu einer Lösung von 2,2 g 1-(4-Chlorbenzyl) homopiperazin und 1 g Triethylamin in 10 ml Methylenchlorid werden bei Zimmertemperatur auf einmal 0,7 g Malonsäuredichlorid zugegeben und das Reaktionsgemisch eine Stunde unter Rückfluß erhitzt. Das Lösungsmittel wird unter vermindertem Druck abgezogen, der Rückstand mit Wasser verrieben, das Rohprodukt mit Methylenchlorid extrahiert und durch Chromatographie an Silikagel gereinigt (Laufmittel Methylenchlorid/Methanol/Hydroxylamin 200 : 5 : 1). Die geeigneten Fraktionen werden gesammelt, das Lösungsmittel verdampft, der Rückstand in Ether gelöst und mit überschüssiger etherischer Salzsäure versetzt ; man erhält die Titelverbindung in kristalliner Form.

Die Verbindungen der allgemeinen Formel I und ihre nicht toxischen, physiologisch veträglichen Säureadditionssalze haben wertvolle pharmakodynamische Eigenschaften.

Sie entfalten insbesondere wegen ihrer starken Hemmung der Mediator-Freisetzung in zahlreichen Zellsystemen entzündungshemmende und antiallergische Eigenschaften in Warmblütern, wie Ratten, und sind deshalb geeignet zur Bekämpfung allergischer Erkrankungen wie allergischem Asthma, Rhinitis, Konjunctivitis, Heufieber, Urticaria, Lebensmittelallergien und dergleichen.

Die Mediator-Freisetzung aus Mastzellen und Basophilen kommt bei vielen allergischen und entzündlichen Erkrankungen vor. Die Aktivität von Substanzen, die die nicht-cytotoxische Exocytose solcher Mediatoren hemmen, kann in in-vitro-Modellen, wie sie die Hemmung der Mediator-Freisetzung aus isolierten Zellsystemen, ausgelöst durch eine Antigen-Antikörperreaktion, darstellt, getestet werden.

In der folgenden Tabelle sind die Daten zusammengestellt, die die biologische Wirksamkeit einiger der erfindungsgemäßen Verbindungen anhand verschiedener Tests zeigen. Folgende Zellsysteme werden in der Tabelle aufgeführt :

RPMC : Peritoneale Mastzellen-Präparationen von Ratten
GPBL : Mit Basophilen angereicherte Leukozyten von Meerschweinchen
HBL : Mit Basophilen angereicherte Leukozyten von Menschen.

Als klinische Standardsubstanzen dienen Theophyllin und Dinatrium-Cromoglycate (DSCG).

(Siehe Tabelle Seite 15 f.)

| Verbindung des Beispiels | Hemmung der Mediator-Freisetzung $IC_{50}$ (μM) * | | |
|---|---|---|---|
| | RPMC | GPBL | HBL |
| 1 | 3 | 40 | 2 |
| 2 | 80 | 20 | 3 |
| 15 | 10 | 50 | 3 |
| 16 | NT | 10 | 0.7 |
| 24 | 0.5 | 9 | 0.7 |
| Theophyllin | >1000 | 200 | 400 |
| DSCG | - | - | >1000 |

* Konzentration der Verbindung, die benötigt wird, die Freisetzung pharmakologische Mediatoren aus den entsprechenden Zellen um 50 % zu hemmen.
NT = nicht getestet.

Für die pharmazeutische Verwendung können die erfindungsgemäßen Verbindungen warmblütigen Tieren topisch, peroral, parenteral, rektal oder durch Inhalation verabreicht werden. Die Verbindungen liegen hierbei als aktive Bestandteile in üblichen Darreichungsformen vor, zum Beispiel in Zusammensetzungen, die im wesentlichen aus einem inerten pharmazeutischen Träger und einer effektiven Dosis des Wirkstoffes bestehen.

Verbindungen der allgemeinen Formel I, die oral gegeben werden, können in Form von Sirupen, Tabletten, Kapseln, Pillen und dergleichen vorliegen. Bevorzugt werden Zusammensetzungen in einer einheitlichen Dosierung oder in einer Form, in der der Patient eine Einzeldosis einnehmen kann. Tabletten, Pulver oder Pastillen können mit jeden für die Formulierung fester pharmazeutischer Zusammensetzungen geeigneten Hilfsstoff vermischt werden. Beispiele solcher Hilfsstoffe sind verschiedene Stärkearten, Lactose, Glucose, Saccharose, Cellulose, Calciumphosphat und Kalk. Die Zusammensetzungen können auch in Form von Kapseln (z. B. aus Gelatin) vorliegen, die den Wirkstoff enthalten oder in Form eines Sirups, einer Lösung oder Suspension. Geeignete flüssige pharmazeutische Trägerstoffe umfassen Ethylalkohol, Glycerin, Kochsalzlösung, Wasser, Propylenglycol oder Sorbitlösung, die mit aromatisierenden oder färbenden Stoffen versetzt sein können.

Die erfindungsgemäßen Verbindungen können auch in Form von Suppositorien für die rektale Anwendung oder in Form einer wässrigen oder nicht wässrigen Injektionslösung, eine Suspension oder eine Emulsion in einer pharmazeutisch unbedenklichen Flüssigkeit vorliegen, wie z. B. sterilem, pyrogenfreiem Wasser oder einem für die parenterale Anwendung geeigneten Öl oder einer Mischung von Flüssigkeiten, die bakteriostatische Mittel, Antioxidantien, Konservierungsmittel Puffersubstanzen oder Lösungen enthalten, die die anzuwendende Lösung in einen isotonischen Zustand versetzen ; ferner können Dickungsmittel, Suspendierhilfsmittel oder andere pharmazeutisch unbedenkliche Zusätze beigefügt werden. Die Anwendung erfolgt in Form von Einzeldosierungen wie Ampullen oder verfügbaren Injektionsgeräten oder in Mehrfachdosierungsbehältern, z. B. einer Flasche, aus der die geeignete Menge entnommen werden kann, oder in fester Form oder in Form eines Konzentrates, das zur Herstellung einer Injektionslösung verwendet werden kann.

Die erfindungsgemäßen Verbindungen können auch in Form von zur Inhalation geeigneten Aerosolen oder Lösungen, die in einem Nebulisator versprüht werden können, oder in Form eines mikrokristallinen Puders zum Einatmen entweder alleine oder in Kombination mit einem inerten Trägerstoff, z. B. Lactose, angewendet werden. Geeignet sind hierfür Partikel der aktiven Verbindungen mit einem Durchmesser von weniger als 20 Mikron, vorzugsweise weniger als 10 Mikron. Gegebenenfalls können hierbei kleinere Mengen anderer Antiallergika, Antiasthmatika und Bronchodilatoren, z. B. Sympatikomymetika wie Isoprenalin, Isoetarin, Metaproterenol, Salbutamol, Phenylephrin, Fenoterol und Ephedrin, ferner Xanthinderivate wie Theophylline und Aminophylline oder Corticosteroide wie Prednisolon und Adrenergika wie ACTH zugefügt werden.

Zur topischen Anwendung an Haut, Nase oder Augen können die erfindungsgemäßen Verbindungen auch in Form einer Salbe, einer Creme, einer Lotion, eines Gels, eines Aerosols oder einer Lösung vorliegen. Topische Lösungen für Nase und Augen können zusätzlich zu den erfindungsgemäßen Verbindungen geeignete Puffersubstanzen, Bakteriostatica, Antioxidantien und Viskositäts- verringernde Mittel, in einem wässrigen Vehicel enthalten.

Beispiele von Mitteln, die die Viskosität erhöhen, sind Polyvinylalkohol, Cellulosederivate, Polyvinylpyrrolidon, Polysorbitanester oder Glyzerin. Geeignete Bakteriostatika schließen ein Benzalkoniumchloride, Thimerosal, Chlorbutanol oder Phenylethylalkohol. Topische Augenpräparate können auch in Form von Salben in einer geeigneten inerten Base, bestehend aus Mineralöl, Petrolatum, Polyethylenglycolen oder Lanolinderivaten, zusammen mit Bakteriostatika vorliegen.

In den oben genannten Formulierungen enthält eine geeignete Dosis von 0,005-500 mg aktiven Wirkstoff. Die wirksame Dosis der erfindungsgemäßen Verbindungen hängt ab von der speziell verwendeten Verbindung, der Verfassung des Patienten und von der Häufigkeit und Art der Anwendung, liegt aber im allgemeinen zwischen 0,0001 mg/kg bis 10 mg/kg Körpergewicht.

Üblicherweise werden den pharmazeutischen Präparaten geschriebene oder gedruckte Hinweise für die betreffende medizinische Anwendung, in diesem Fall als Antiallergikum für die Prophylaxe und die Behandlung für z. B. Asthma, Heufieber, Rhinitis oder allergisches Eczem, beigefügt.

Für die Herstellung pharmazeutischer Präparate werden die Verbindungen der allgemeinen Formel I in üblicher Weise mit geeigneten pharmazeutischen Trägerstoffen und Aromastoffen, Durftstoffen und Farbstoffen gemischt und beispielsweise zu Tabletten verpreßt, in Kapseln eingefüllt, oder mit Zusatz entsprechender Adjuvantien, in Wasser oder in einem Öl, beispielsweise Maisöl, suspendiert oder gelöst.

Die erfindungsgemäßen Verbindungen können oral oder parenteral in flüssiger oder fester Form angewendet werden. Für Injektionslösungen wird bevorzugt Wasser verwendet, das diejenigen Stabilisierungsmittel, Lösungsvermittler und/oder Puffersubstanzen enthält, die gewöhnlich für Injektionslösungen benutzt werden. Zusatzstoffe dieser Art umfassen beispielsweise Tartrat-, Citrat- und Acetat-Puffer, Ethanol, Propylenglycol, Polyethylenglycol, Komplexbildner (z. B. EDTA) Antioxidantien (z. B. Natriumbisulfit, Natriummetabisulfit oder Ascorbinsäure) hochmolekulare Polymere (wie beispielsweise flüssige Polyethylenoxide) zur Viskositätsregelung und Polyethylenderivate von Sorbitanhydriden.

Gewünschtenfalls können Konservierungsstoffe zugefügt werden, z. B. Benzoelsäure Methyl- oder Propyl-Paraben Benzalkoniumchlorid oder quarternäre Ammoniumverbindungen.

Zur Verwendung geeignete feste Trägerstoffe sind beispielsweise Stärke, Lactose, Mannit, Methylcellulose, mikrokristalline Cellulose, Talcum, Kieselsäure, Dicalciumphosphat aund hochmolekulare Polymere (beispielsweise Polyethylenglycol).

Präparate für die orale Anwendung können gewünschtenfalls Geruchs- und/oder Süßungsmittel enthalten.

Für die topische Anwendung können die erfindungsgemäßen Verbindungen auch in Form von Lösungen, Pudern oder Salben angewendet werden; hierzu werden sie z. B. mit physiologisch verträglichen Verdünnungsmitteln oder üblichen Salbengrundlagen vermischt.

Die folgenden Beispiele beschreiben einige pharmazeutische Zusammensetzungen, die eine erfindungsgemäße Verbindung als aktiven Wirkstoff enthalten. Falls nicht besonders darauf hingewiesen wird, handelt es sich bei den Teilen um Gewichtsteile.

Beispiel 36

Tabletten

Die Tabletten enthält folgende Bestandteile :

| | |
|---|---|
| 1,3-Bis[4-(4-chlorbenzyl)-1-piperazinyl]propan tetrahydrochlorid | 0,010 Teile |
| Stearinsäure | 0,010 Teile |
| Glukose | 1,890 Teile |
| | 1,910 Teile |

Herstellung :

Die Bestandteile werden in üblicher Weise gemischt und die Mischung in Tabletten von 1,91 g verpreßt ; jede Tablette enthält 10 mg Wirkstoff.

Beispiel 37

Salbe

Die Salbe ist wie folgt zusammengesetzt :

| | |
|---|---|
| 1,3-Bis[4-(4-chlorbenzyl)-1-piperazinyl]-2-hydroxypropan | 2,000 Teile |
| Rauchende Salzsäure | 0,011 Teile |
| Natriumpyrosulfit | 0,050 Teile |
| Cetylalkohol/Stearylalkohol (1:1) | 20,000 Teile |
| Weiße Vaseline | 5,000 Teile |
| Synthetisches Bergamottöl | 0,075 Teile |
| Destilliertes Wasser | ad 100,000 Teile |

Herstellung :

Die Bestandteile werden in üblicher Weise zu einer Salbe vermischt ; 100 g der Salbe enthalten 2,0 g des aktiven Wirkstoffs.

## Beispiel 38

Inhalationsaerosol

Das Aerosol ist aus folgenden Bestandteilen zusammengesetzt :

| | |
|---|---|
| 1,3-Bis[4-(4-chlorbenzyl)-1-piperazinyl]-1,3-dioxopropan-dihydrochlorid-monohydrat | 1,00 Teile |
| Sojalecitnin | 0,20 Teile |
| Treibgasmischung (Freon 11, 12 und 14) | ad 100,00 Teile |

Herstellung :

Die Bestandteile werden in üblicher Weise vermischt und in mit einem Dosierventil versehene Aerosolbehälter gefüllt, wobei das Dosierventil 0,5-2,0 mg des aktiven Wirkstoffs pro Betätigung des Ventils abgibt.

## Beispiel 39

Injektionslösung

Die Lösung setzt sich aus folgenden Bestandteilen zusammen ,

| | |
|---|---|
| 1,3-Bis[4-(4-chlorphenethyl)-1-piperazinyl]propan-dihydrochlorid | 5,0 Teile |
| Natriumpyrosulfit | 1,0 Teile |
| EDTA-Natriumsalz | 0,5 Teile |
| Natriumchlorid | 8,5 Teile |
| Doppelt destilliertes Wasser | ad 1000,0 Teile |

Herstellung :

Die einzelnen Bestandteile werden in einer genügenden Menge doppeltdestilliertem Wasser gelöst, die Lösung bis zur angegebenen Konsentration mit weiterem doppeltdestilliertem Wasser verdünnt, die erhaltene Lösung durch Filtrieren von suspendierten Partikeln befreit und das Filtrat unter aseptischen Bedingungen in 1 ml Ampullen gefüllt, die anschließend sterilisiert und verschlossen werden. Jeder Ampulle enthält 5 ml des Wirkstoffs.

## Beispiel 40

Topische Lösung (für Augen- oder Nasentropfen)

Die Lösung ist aus folgenden Bestandteilen zusammengesetzt :

| | |
|---|---|
| 1,3-Bis[4-(4-chlorbenzyl)-1-piperazinyl]-2-hydroxypropan tetrahydrochlorid | 0,020 Teile |
| Dinatriumhydrogenphosphat | 0,758 Teile |
| Natriumdihydrogenphosphat | 0,184 Teile |
| Natriumchlorid | 0,365 Teile |
| Polyvinylalkohol | 3,500 Teile |
| Benzalkoniumchlorid | 0,010 Teile |
| Destilliertes Wasser | ad 100,000 Teile |

Herstellung :

Die Bestandteile werden in üblicher Art gelöst. Die Lösung wird filtriert, wobei die Lösung für Augentropfen eine sterile Filtration erfordert. Jeder ml Lösung enthält 0,2 mg Wirkstoff.

Die in den Beispielen 36 bis 40 genannten Wirkstoffe können durch jeden anderen von der allgemeinen Formel I umfaßten Wirkstoff oder einen seiner nicht toxischen pharmazeutisch verträglichen Säureadditionssalze ersetzt werden. Ebenso kann der Anteil an Wirkstoff innerhalb der oben angegebenen Dosierung variiert werden, und auch Menge und Art der inerten pharmazeutischen Trägerstoffe können aufgrund spezieller Erfordernisse abgeändert werden.

## Patentansprüche

1. Bis-(Piperazinyl-bzw. Homopiperazinyl)alkane der allgemeinen Formel (I)

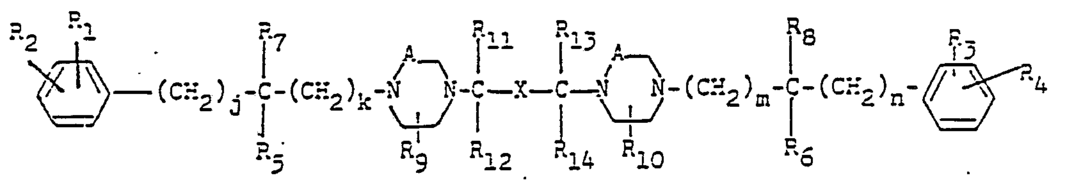 (I)

worin

$R_1$, $R_2$, $R_3$ und $R_4$, die gleich oder verschieden sein können, Wasserstoff, eine Alkylgruppe mit 1 bis 4 Kohlenstoffatomen, die Hydroxygruppe, eine Alkoxygruppe mit 1 bis 4 Kohlenstoffatomen, eine Acyloxygruppe mit 1 bis 4 Kohlenstoffatomen, Halogen, Trihalogenmethyl, Di-$C_{1-4}$-alkylamino, $C_{1-4}$-Alkoxycarbonyl, Nitro, Cyano oder Acyl mit 1 bis 3 Kohlenstoffatomen;

$R_5$ und $R_6$, die gleich oder verschieden sein können, Wasserstoff, Methyl, Hydroxy, Carboxy, $C_{1-4}$-Alkoxycarbonyl, Hydroxymethyl, Phenyl oder p-Chlorphenyl;

$R_7$ und $R_8$ Wasserstoff oder Methyl

j und k Indices von 0-3, insgesamt jedoch nicht mehr als 4;

m and n Indices von 0-3, insgesamt jedoch nicht mehr als 4;

A $-CH_2-$ oder $-CH_2-CH_2-$; oder

$R_5$ und $R_7$ zusammen oder $R_6$ und $R_8$ zusammen Oxo, mit der Maßgabe, daß k oder m von 0 verschieden sind; oder

$R_5$ und $R_7$ zusammen und $R_6$ und $R_8$ zusammen Oxo, mit der Maßgabe, daß k und m von 0 verschieden sind; oder

$R_9$ und $R_{10}$, die gleich oder verschieden sein können, wasserstoff, oder einen bis vier Methylsubstituenten an den Kohlenstoffatomen eines Piperazinrings (A = $-CH_2-$);

$R_{11}$, $R_{12}$, $R_{13}$ und $R_{14}$, die gleich oder verschieden sein können, Wasserstoff oder Methyl oder

$R_{11}$ und $R_{12}$ zusammen und/oder $R_{13}$ und $R_{14}$ zusammen Oxo; und

X eine Alkylenkette mit 1 bis 2 Kohlenstoffatomen, die gegebenenfalls durch Hydroxy substituiert ist, bedeutet, mit der Maßgabe, daß wenn A = $-CH_2-$, $R_1$ bis $R_{14}$ Wasserstoff, und j, k, m und n 0 darstellen, X nicht 1,2-ethylendiol bedeuten kann und deren physiologisch unbedenklichen Säureadditionssalze.

2. Eine Verbindung gemäß Anspruch 1 der allgemeinen Formel (II)

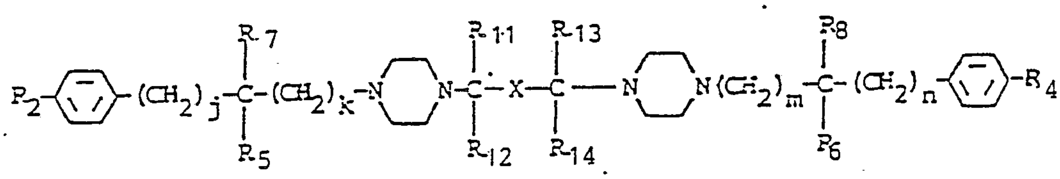 (II)

worin

j, k, m und n 0, 1, oder 2;

$R_2$ und $R_4$ Wasserstoff, Chlor, Methyl oder ($C_1$-$C_4$) Alkoxy;

$R_5$, $R_6$, $R_7$, $R_8$ sowie $R_{11}$, $R_{12}$, $R_{13}$, $R_{14}$ und X die in Anspruch 1 angegeben Bedeutung besitzen, und deren physiologisch verträgliche Säureadditionssalze.

3. Eine Verbindung gemäß Anspruch 1 der allgemeinen Formel (III)

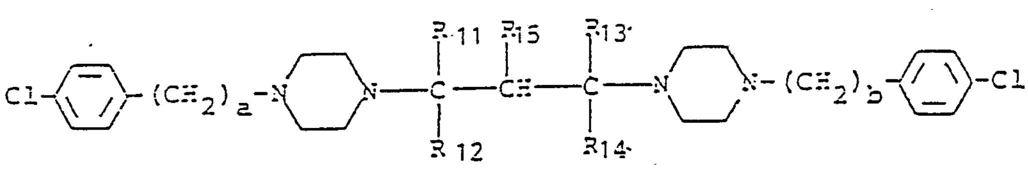 (III)

worin

$R_{11}$ und $R_{12}$ Wasserstoff oder zusammen Oxo ;

$R_{15}$ Wasserstoff oder Hydroxy ;

$R_{13}$ und $R_{14}$ Wasserstoff oder zusammen Oxo ; und a und b 1, 2, 3 oder 4 bedeuten und deren physiologisch verträglichen Säureadditionssalze.

4. 1,3-Bis[4-(4-chlorbenzyl)-1-piperazinyl]propan und dessen physiologisch verträglichen Säureadditionssalze.

5. 1,3-Bis[4-(4-chlorbenzyl)-1-piperazinyl]-2-hydroxy-propan und dessen physiologisch verträglichen Säureadditionssalze.

6. 1,3-Bis[4-(4-chlorbenzyl)-1-piperazinyl]-1,3-dioxo-propan und dessen physiologisch verträglichen Säureadditionssalze.

7. 1,3-Bis[4-(4-chlorphenethyl)-1-piperazinyl]propan und dessen physiologisch verträglichen Säureadditionssalze.

8. 1,3-Bis[4-(3-{4-chlorphenyl}-propyl)-1-piperazinyl]-propan und dessen physiologisch verträglichen Säureadditionssalze.

9. Verfahren zur Herstellung von Bis-(piperazinyl- bzw. Homopiperazinyl) alkanen der allgemeinen Formel (I) gemäß Anspruch 1, dadurch gekennzeichnet, daß man

a) zur Herstellung symmetrischer Verbindungen der allgemeinen Formel I, eine Verbindung der allgemeinen Formel (IV)

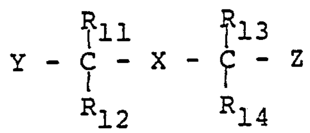

(IV)

worin $R_{11}$, $R_{12}$, $R_{13}$, $R_{14}$ und X die in Anspruch 1 angegebene Bedeutung haben und Y und Z reaktive Gruppen bedeuten, mit einer Verbindung der allgemeinen Formel (V)

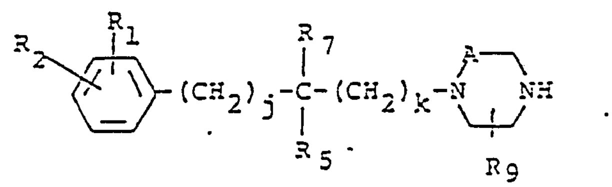

(V)

worin $R_1$, $R_2$, $R_5$, $R_7$, $R_9$, j, k und A die in Anspruch 1 angegebene Bedeutung besitzen, umsetzt, oder daß man

b) eine Verbindung der allgemeinen Formel (VI)

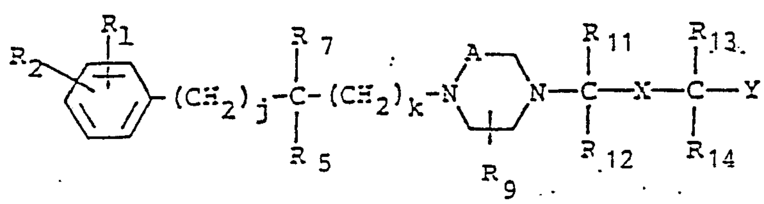

(VI)

worin $R_1$, $R_2$, $R_5$, $R_7$, $R_9$, $R_{11}$, $R_{12}$, $R_{13}$, $R_{14}$, X, j, k und A die in Anspruch 1 angegebene Bedeutung besitzen, und Y eine reaktive Gruppe bedeutet mit einer Verbindung der allgemeinen Formel (VII)

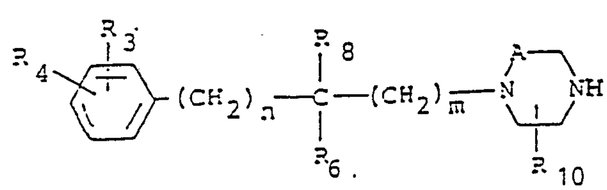

(VII)

worin $R_3$, $R_4$, $R_6$, $R_8$, $R_{10}$, m, n und A die oben angegebene Bedeutung haben, umsetzt, oder daß man

c) zur Herstellung solcher Endprodukte der allgemeinen Formel (I), die mit Bezug auf die zentrale Gruppe X symmetrisch sind, eine Verbindung der allgemeinen Formel (VIII)

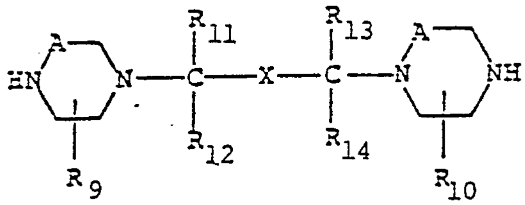

(VIII)

worin $R_9$, $R_{10}$, $R_{11}$, $R_{12}$, $R_{13}$, $R_{14}$, X und A die in Anspruch 1 angegebene Bedeutung besitzen, mit einer Verbindung der allgemeinen Formel (IX)

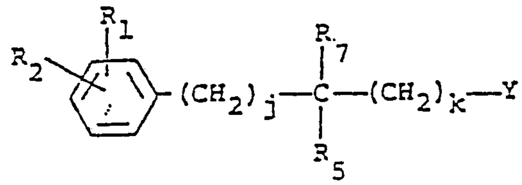

(IX)

worin $R_1$, $R_2$, $R_5$, $R_7$, Y, j und k die in Anspruch 1 angegebene Bedeutung besitzen, umsetzt, oder daß man

d) zur Herstellung solcher Endprodukte der allgemeinen Formel (I), in denen X eine Carbinolgruppe bedeutet, eine Verbindung der allgemeinen Formel (X)

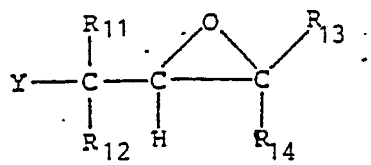

(X)

worin

$R_{11}$, $R_{12}$, $R_{13}$ und $R_{14}$ die in Anspruch 1 angegebene Bedeutung besitzen und

Y die gleiche Bedeutung hat wie in Formel (IV), mit einer Verbindung der allgemeinen Formel (VII) umsetzt,

und daß man gegebenenfalls ein nach einem der Verfahren a-d hergestelltes Endprodukt der allgemeinen Formel I in an sich bekannter Weise in ein physiologisch unbedenkliches Säureadditionssalz überführt.

10. Pharmazeutische Präparate, enthaltend als Wirkstoff eine Verbindung der allgemeinen Formel (I)

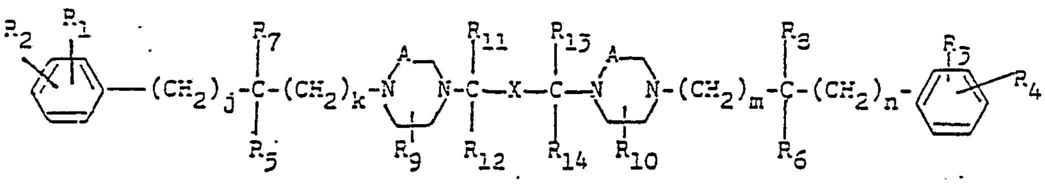

(I)

worin

$R_1$, $R_2$, $R_3$ und $R_4$, die gleich oder verschieden sein können, Wasserstoff, eine Alkylgruppe mit 1 bis 4 Kohlenstoffatomen, die Hydroxygruppe, eine Alkoxygruppe mit 1 bis 4 Kohlenstoffatomen, eine Acyloxygruppe mit 1 bis 4 Kohlenstoffatomen, Halogen, Trihalogenmethyl, Di-$C_{1-4}$-alkylamino, $C_{1-4}$-Alkoxycarbonyl, Nitro, Cyano oder Acyl mit 1 bis 3 Kohlenstoffatomen ;

$R_5$ und $R_6$, die gleich oder verschieden sein können, Wasserstoff, Methyl, Hydroxy, Carboxy, $C_{1-4}$-Alkoxycarbonyl, Hydroxymethyl, Phenyl oder p-Chlorphenyl ;

$R_7$ und $R_8$ Wasserstoff oder Methyl

j und k Indices von 0-3, insgesamt jedoch nicht mehr als 4 ;

m und n Indices von 0-3, insgesamt jedoch nicht mehr als 4 ;

A $-CH_2-$ oder $-CH_2-CH_2-$ ; oder

$R_5$ und $R_7$ zusammen oder $R_6$ und $R_8$ zusammen Oxo, mit der Maßgabe, daß k oder m von 0 verschieden sind ; oder

$R_5$ und $R_7$ zusammen und $R_6$ und $R_8$ zusammen Oxo mit der Maßgabe, daß k und m von 0 verschieden sind ;

$R_9$ und $R_{10}$, die gleich oder verschieden sein können, Wasserstoff, oder einen bis vier Methylsubstituenten an den Kohlenstoffatomen eines Piperazinrings ($A = -CH_2-$) ;

$R_{11}$, $R_{12}$, $R_{13}$ und $R_{14}$, die gleich oder verschieden sein können, mit Wasserstoff oder Methyl oder

$R_{11}$ und $R_{12}$ zusammen und/oder $R_{13}$ und $R_{14}$ zusammen Oxo ; und

X eine Alkylenkette mit 1 bis 2 Kohlenstoffatomen, die gegebenenfalls durch Hydroxy substituiert ist, bedeutet,
oder eine ihrer physiologisch unbedenklichen Säureadditionssalze, in Kombination mit üblichen Hilfs- und/oder Trägerstoffen.

11. Pharmazeutische Präparate, enthaltend als Wirkstoff ein Verbindung gemäß Anspruch 10 in Kombination mit weiteren pharmakodynamisch wirksamen Stoffen sowie üblichen Hilfs- und/oder Trägerstoffen.

12. Verfahren zur Herstellung von pharmazeutischen Präparaten gemäß Anspruch 10, dadurch gekennzeichnet, daß man eine Verbindung gemäß Anspruch 10 mit üblichen galenischen Hilfs- und/oder Trägerstoffen zur üblichen pharmazeutischen Anwendungsformen verarbeitet.

13. Verfahren zur Herstellung von pharmazeutischen Präparaten gemäß Anspruch 11, dadurch gekennzeichnet, daß man eine Verbindung gemäß Anspruch 10 in Kombination mit anderen pharmakodynamisch wirksamen Stoffen, sowie üblichen Hilfs- und/oder Trägerstoffen zu üblichen pharmazeutischen Anwendungsformen verarbeitet.

14. Die Verwendung einer Verbindung gemäß Anspruch 10 zur Herstellung von pharmazeutischen Präparaten zur Behandlung von allergischen Reaktionen und Entzündungen bei Warmblütern.

**Claims**

1. Bis-(piperazinyl- or homopiperazinyl) alkanes of the general formula I

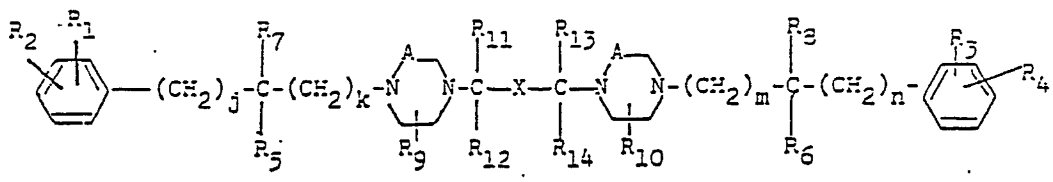

(I)

wherein

$R_1$, $R_2$, $R_3$ and $R_4$ which may be identical to or different from each other, are each hydrogen, alkyl having 1 to 4 carbon atoms, hydroxyl, alkoxy having 1 to 4 carbon atoms, alkanoyloxy having up to 4 carbon atoms, halogen, trihalomethyl, di-$C_{1-4}$ alkylamino, $C_{1-4}$ alkoxycarbonyl, nitro, cyano or acyl having 1 to 3 carbon atoms ;

$R_5$ and $R_6$, which may be identical to or different from each other, are each hydrogen, methyl, hydroxyl, carboxyl, $C_{1-4}$ alkoxycarbonyl, hydroxymethyl, phenyl or p-chlorophenyl,

$R_7$ and $R_8$ are each hydrogen or methyl ;

j and k are integers from 0 to 3, their sum being no more than 4 ;

m and n are integers from 0 to 3, their sum being no more than 4 ;

A is $-CH_2-$ or $-CH_2CH_2-$ ; or

$R_5$ and $R_7$ together or $R_6$ and $R_8$ together are oxo, provided k or m is other than 0 ; or

$R_5$ and $R_7$ together and $R_6$ and $R_8$ together are oxo, provided k or m are other than 0 ;

$R_9$ and $R_{10}$, which may be the same or different, represent hydrogen or from one to four methyl substituents on the carbon atoms of the piperazine ring ($A = -CH_2-$) ;

$R_{11}$, $R_{12}$, $R_{13}$ and $R_{14}$, which may be identical to or different from each other, are each hydrogen or methyl ; or

$R_{11}$ and $R_{12}$ together and/or $R_{13}$ and $R_{14}$ together are oxo ; and

X is alkylene of 1 to 2 carbon atoms, optionally hydroxy-substituted with the proviso that when $A = -CH_2-$, $R_1$ to $R_{14}$ are hydrogen and j, k, m and n are each zero, then X cannot be 1,2-ethylenediol, and physiologically acceptable acid addition salts thereof.

2. A compound according to claim 1 of formula II

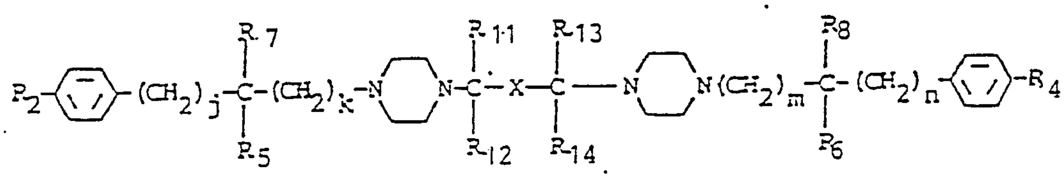

(II)

wherein

j, k, m and n are each 0, 1 or 2 ;

$R_2$ and $R_4$ are $-H$, $-Cl$, $-CH_3$ or $C_{1-4}$ alkoxy ;

$R_5$, $R_6$, $R_7$, $R_8$ as well as $R_{11}$, $R_{12}$, $R_{13}$, $R_{14}$ and X are as set forth in claim 1, and physiologically acceptable acid addition salts thereof.

3. A compound according to claim 1 of formula III

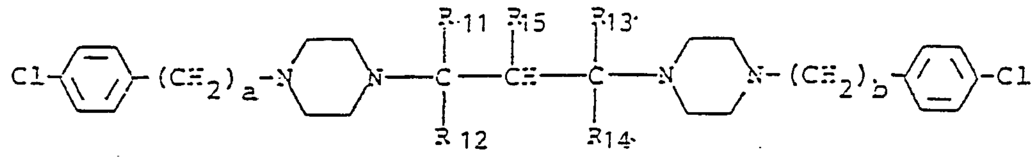

(III)

wherein

$R_{11}$ and $R_{12}$ are H, or together are oxo ;

$R_{15}$ is H or OH ;

$R_{13}$ and $R_{14}$ are H, or together are oxo ; and

a and b are each 1, 2, 3 or 4, and physiologically acceptable acid addition salts thereof.

4. 1,3-bis[4-(4-chlorobenzyl)-1-piperazinyl] propane and the physiologically acceptable acid addition salts thereof.

5. 1,3-bis[4-(4-chlorobenzyl)-1-piperazinyl]-2-hydroxy-propane and the physiologically acceptable acid addition salts thereof.

6. 1,3-bis[4-(4-chlorobenzyl)-1-piperazinyl]-1,3-dioxo-propane and the physiologically acceptable acid addition salts thereof.

7. 1,3-bis[4-(4-chlorophenethyl)-1-piperazinyl]propane and the physiologically acceptable acid addition salts thereof.

8. 1,3-bis[4-(3-[4-chlorophenyl]propyl)-1-piperazinyl]propane and the physiologically acceptable acid addition salts thereof.

9. A process for the preparation of bis-(piperazinyl- or homopiperazinyl) alkanes of general formula I according to claim 1, characterised in that

a) to prepare symmetric compounds of formula I a compound of general formula IV

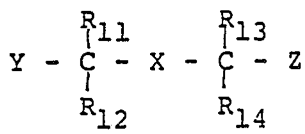

(IV)

wherein $R_{11}$, $R_{12}$, $R_{13}$, $R_{14}$ and X are as defined in claim 1 and Y and Z are reactive groups is reacted with a compound of formula V

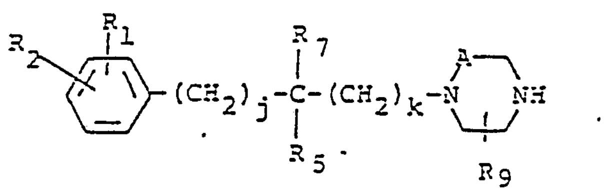

(V)

wherein $R_1$, $R_2$, $R_5$, $R_7$, $R_9$, j, k and A are as defined in claim 1 ;

b) reacting a compound of formula VI

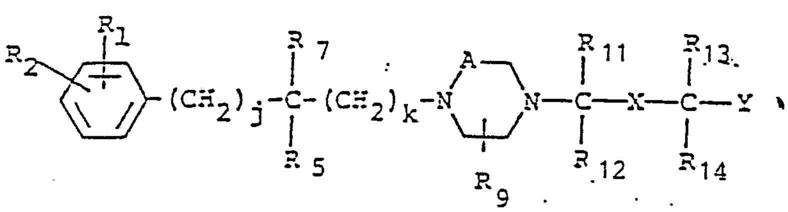

(VI)

wherein

$R_1$, $R_2$, $R_5$, $R_7$, $R_9$, $R_{11}$, $R_{12}$, $R_{13}$, $R_{14}$, X, j, k and A are as defined in claim 1 and Y is a reactive group with a compound of formula VII

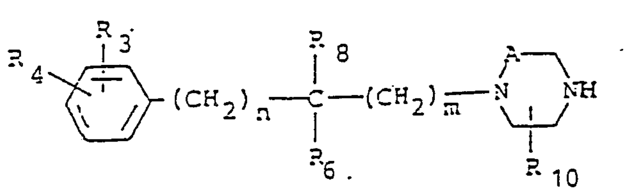

(VII)

wherein $R_3$, $R_4$, $R_6$, $R_8$, $R_{10}$, m, n and A are as defined in claim 1.

c) (to prepare compounds of formula I, which is symmetrical about central group X) reacting a compound of formula VIII

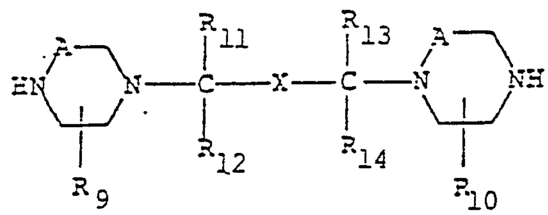

(VIII)

wherein $R_9$, $R_{10}$, $R_{11}$, $R_{12}$, $R_{13}$, $R_{14}$, X and A are as defined in claim 1 with a compound of formula IX

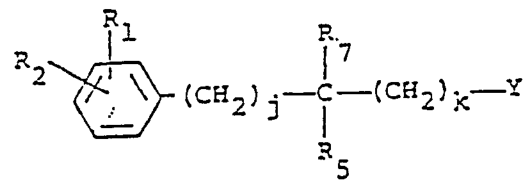

(IX)

wherein $R_1$, $R_2$, $R_5$, $R_7$, Y, j and k are as defined in claim 1 or

d) (to prepare compounds of formula I wherein X is a carbinol moiety) reacting a compound of formula X

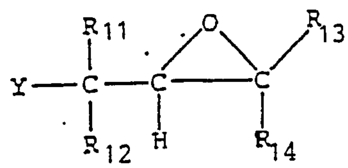

(X)

wherein $R_{11}$, $R_{12}$, $R_{13}$ and $R_{14}$ are as defined in claim 1 and Y has the same meaning as in formula IV, with a compound of formula VII and optionally converting a compound of formula I produced by one of the processes a) to d) into a physiologically acceptable acid addition salt thereof in a manner know per se.

10. A pharmaceutical preparation, containing as active ingredient a compound of the general formula

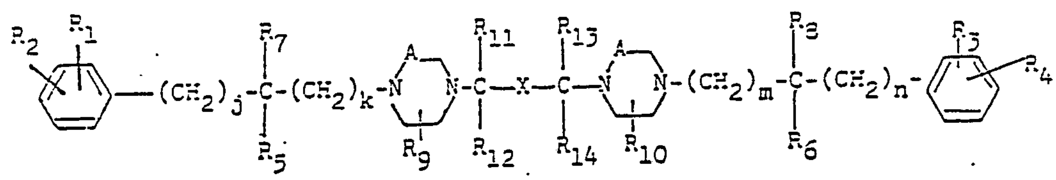

(I)

in which

$R_1$, $R_2$, $R_3$ and $R_4$ can be the same or different and are hydrogen, an alkyl group containing 1 to 4 carbon atoms, hydroxy, an alkoxy group containing 1 to 4 carbon atoms, an acyloxy group containing 1 to 4 carbon atoms, halogen, trihalomethyl, di-($C_{1-4}$alkyl) amino, $C_{1-4}$alkoxycarbonyl, nitro, cyano or acyl containing 1 to 3 carbon atoms,

$R_5$ and $R_6$ can be the same or different and are hydrogen, methyl, hydroxy, carboxy, $C_{1-4}$ alkoxycarbonyl, hydroxymethyl, phenyl or p-chlorophenyl ;

$R_7$ and $R_8$ are hydrogen or methyl ;

j and k are integers from 0 to 3, their sum being no more than 4 ;

m and n are integers from 0 to 3, their sum being not more than 4 ;

A is $-CH_2-$ or $-CH_2CH_2-$ ; or

$R_5$ and $R_7$ together or $R_6$ and $R_8$ together are oxo, with the proviso that k or m is not zero, or

$R_5$ and $R_7$ together and $R_6$ and $R_8$ together are oxo provided that k and m are other than 0 ;

$R_9$ and $R_{10}$ are the same or different, and are hydrogen, or one or more methyl substituents on the carbon atoms of a piperazinyl ring (A = $-CH_2)-$ ;

$R_{11}$, $R_{12}$, $R_{13}$ and $R_{14}$ are the same or different, are hydrogen or methyl or

$R_{11}$ and $R_{12}$ together and/or $R_{13}$ and $R_{14}$ together are oxo and

X is an alkyl chain with 1 to 2 carbon atoms optionally substituted by hydroxy,

or a physiologically acceptable acid addition salt thereof, in combination with a conventional carrier or excipient.

11. A pharmaceutical preparation containing as an active ingredient a compound according to claim 10, in combination with a further pharmacologically active substance and a conventional carrier or excipient.

12. A process for the preparation of a pharmaceutical preparation according to claim 10, characterised in that a compound according to claim 10 and a conventional galenic carrier or excipient are worked into a conventional pharmaceutical form.

13. A process for the preparation of a pharmaceutical preparation according to claim 11, characterised in that a compound according to claim 10 in combination with another pharmacologically active substance, and a conventional carrier or excipient, are worked into a conventional pharmaceutical form.

14. The use of a compound according to claim 10, in the production of a pharmaceutical preparation for the treatment of allergic reactions and inflammations in warm-blooded animals.

**Revendications**

1. Bis-(pipérazinyl- ou homopipérazinyl) alcanes répondant à la formule générale I

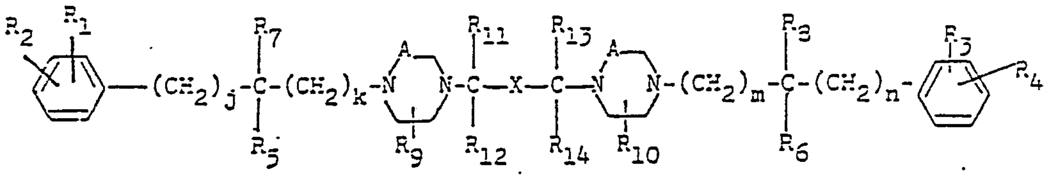
(I)

dans laquelle

$R_1$, $R_2$, $R_3$ et $R_4$, qui peuvent être identiques ou différents, désignent un atome d'hydrogène, un groupe alkyle en $C_1$ à $C_4$, le groupe hydroxy, un groupe alcoxy en $C_1$ à $C_4$, un groupe alcyloxy en $C_1$ à $C_4$, un atome d'halogène, un groupe trihalogénométhyle, un groupe di-(alkyle en $C_1$ à $C_4$) amino, (alcoxy en $C_1$ à $C_4$) carbonyle, nitro, cyano ou acyle en $C_1$ à $C_3$ ;

$R_5$ et $R_6$, qui peuvent être identiques ou différents, désignent un atome d'hydrogène, un groupe méthyle, hydroxy, carboxy, (alcoxy en $C_1$ à $C_4$) carbonyle, hydroxyméthyle, phényle ou p-chlorophényle ;

$R_7$ et $R_8$ désignent un atome d'hydrogène ou un groupe méthyle ;

j et k désignent des indices de 0 à 3, mais dont le total n'est pas supérieur à 4 ;

m et n désignent des indices de 0 à 3, mais dont le total n'est pas supérieur à 4 ;

A désigne $-CH_2-$ ou $-CH_2-CH_2-$ ; ou

$R_5$ et $R_7$ ou $R_6$ et $R_8$ désignent ensemble un groupe oxo, sous réserve que k ou m soit différent de 0 ; ou

$R_5$ et $R_7$ et $R_6$ et $R_8$ désignent ensemble un groupe oxo, sous réserve que k et m soient différents de 0 ; ou

$R_9$ et $R_{10}$, qui peuvent être identiques ou différents, désignent un atome d'hydrogène ou un à quatre substituants méthyle sur l'atome de carbone d'un cycle pipérazine ($A = -CH_2-$) ;

$R_{11}$, $R_{12}$, $R_{13}$ et $R_{14}$, qui peuvent être identiques ou différents, désignent un atome d'hydrogène ou un groupe méthyle ou

$R_{11}$ et $R_{12}$ et/ou $R_{13}$ et $R_{14}$ désignent ensemble un groupe oxo ; et

X désigne une chaîne alkylène en $C_1$ à $C_2$, qui est substituée le cas échéant par un groupe hydroxy, sous réserve que lorsque $A = -CH_2-$, $R_1$ à $R_{14}$ désignent un atome d'hydrogène et j, k, m et n désignent 0, X ne peut pas désigner le 1,2-éthylène-diol, et leurs sels d'addition aux acides physiologiquement acceptables.

2. Composé suivant la revendication 1, répondant à la formule II

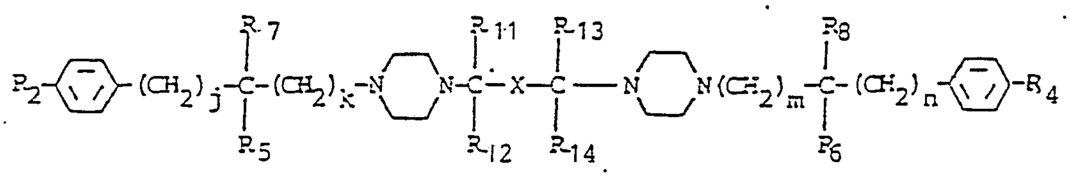
(II)

dans laquelle

j, k, m et n sont 0, 1 ou 2 ;

$R_2$ et $R_4$ désignent un atome d'hydrogène, un atome de chlore, un groupe méthyle ou alcoxy en $C_1$ à $C_4$ ;

$R_5$, $R_6$, $R_7$, $R_8$ ainsi que $R_{11}$, $R_{12}$, $R_{13}$, $R_{14}$ et X ont les significations indiquées dans la revendication 1.

3. Composé suivant la revendication 1, répondant à la formule générale III

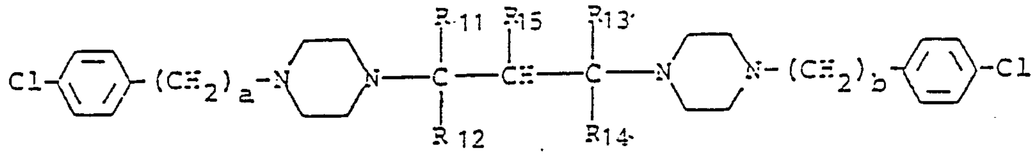

(III)

dans laquelle

$R_{11}$ et $R_{12}$ désignent un atome d'hydrogène ou forment ensemble un groupe oxo ;
$R_{15}$ désigne un atome d'hydrogène ou un groupe hydroxy ;
$R_{13}$ et $R_{14}$ désignent un atome d'hydrogène ou forment ensemble un groupe oxo ; et
a et b désignent 1, 2, 3 ou 4

et ses sels d'addition aux acides pharmaceutiquement acceptables.

4. 1,3-bis[4-(4-chlorobenzyl)-1-pipérazinyl] propane et ses sels d'addition aux acides pharmaceutiquement acceptables.

5. 1,3-bis[4-(4-chlorobenzyl)-1-pipérazinyl]-2-hydroxypropane et ses sels d'addition aux acides pharmaceutiquement acceptables.

6. 1,3-bis[4-(4-chlorobenzyl)-1-pipérazinyl]-1,3-dioxopropane et ses sels d'addition aux acides pharmaceutiquement acceptables.

7. 1,3-bis[4-(4-chlorophénéthyl)-1-pipérazinyl]-propane et ses sels d'addition aux acides pharmaceutiquement acceptables.

8. 1,3-bis[4-(3-{4-chlorophényl}-propyl)-1-pipérazinyl]propane et ses sels d'addition aux acides pharmaceutiquement acceptables.

9. Procédé de préparation de bis-(pipérazinyl- ou homopipérazinyl) alcanes répondant à la formule générale I suivant la revendication 1, caractérisé en ce que

a) pour la préparation de composés symétriques répondant à la formule générale I, on fait réagir un composé répondant à la formule générale IV,

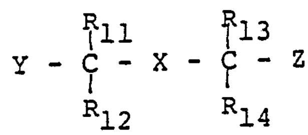

(IV)

dans laquelle $R_{11}$, $R_{12}$, $R_{13}$, $R_{14}$ et X ont les significations indiquées dans la revendication 1 et Y et Z désignent des groupes réactifs, avec un composé répondant à la formule générale V

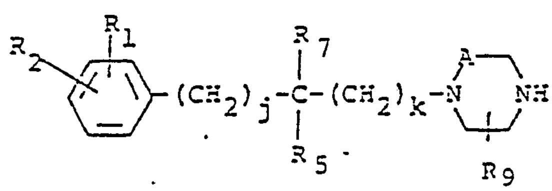

(V)

dans laquelle $R_1$, $R_2$, $R_5$, $R_7$, $R_9$, j, k et A possèdent les significations indiquées dans la revendication 1, ou en ce que

b) on fait réagir un composé répondant à la formule générale VI

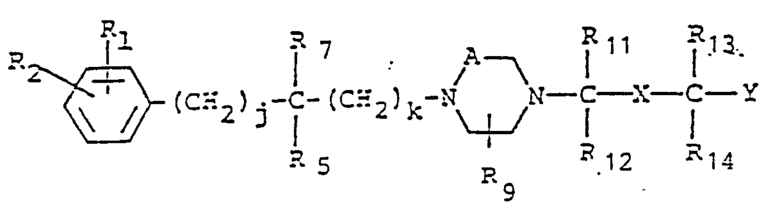

(VI)

dans laquelle $R_1$, $R_2$, $R_5$, $R_7$, $R_9$, $R_{11}$, $R_{12}$, $R_{13}$, $R_{14}$, X, j, k, et A possèdent les significations indiquées dans la revendication 1, et Y désigne un groupe réactif, avec un composé répondant à la formule générale VII

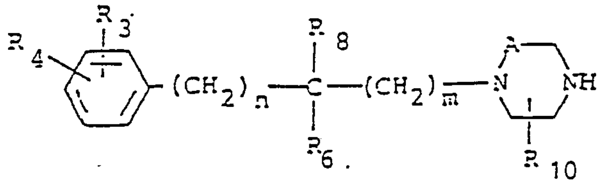

(VII)

dans laquelle $R_3$, $R_4$, $R_6$, $R_8$, $R_{10}$, m, n et A ont les significations indiquées ci-dessus, ou en ce que

c) pour la préparation des produits finaux répondant à la formule générale I qui sont symétriques par rapport au groupe central X, on fait réagir un composé répondant à la formule générale VIII

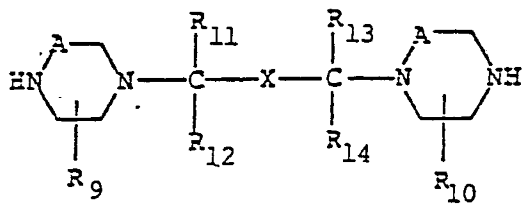

(VIII)

dans laquelle $R_9$, $R_{10}$, $R_{11}$, $R_{12}$, $R_{13}$, $R_{14}$, X et A possèdent les significations indiquées dans la revendication 1, avec un composé répondant à la formule générale IX

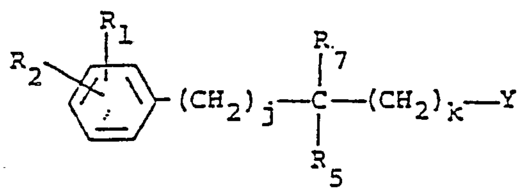

(IX)

dans laquelle $R_1$, $R_2$, $R_5$, $R_7$, Y, j et k possèdent les significations indiquées dans la revendication 1, ou en ce que

d) pour la préparation des produits finaux répondant à la formule générale I, dans lesquels X désigne un groupe carbinol, on fait réagir un composé répondant à la formule X

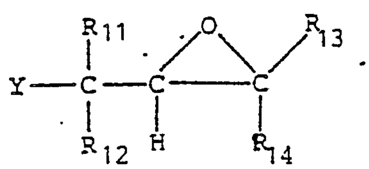

(X)

dans laquelle $R_{11}$, $R_{12}$, $R_{13}$ et $R_{14}$ ont les significations indiquées dans la revendication 1 et Y a la même signification que dans la formule IV, avec un composé répondant à la formule générale VII, et en ce qu'on transforme le cas échéant d'une manière connue en soi un produit final répondant à la formule générale I, préparé suivant l'un des procédés a à d, en un sel d'addition aux acides physiologiquement inoffensif.

10. Préparations pharmaceutiques contenant comme principe actif un composé répondant à la formule générale I

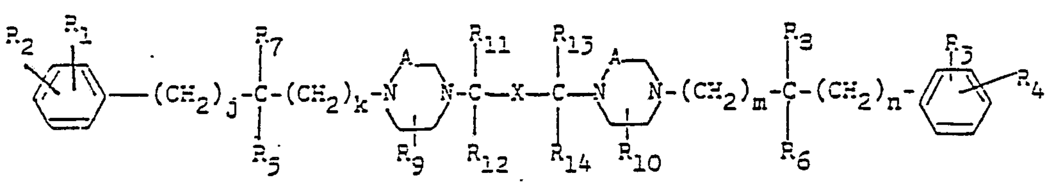

(I)

dans laquelle

$R_1$, $R_2$, $R_3$ et $R_4$, qui peuvent être identiques ou différents, désignent un atome d'hydrogène, un groupe alkyle en $C_1$ à $C_4$, le groupe hydroxy, un groupe alcoxy en $C_1$ à $C_4$, un groupe alcyloxy en $C_1$ à $C_4$, un atome d'halogène, un groupe trihalogénométhyle, di-(alkyle en $C_1$ à $C_4$) amino, (alkoxy en $C_1$ à $C_4$) carbonyle, nitro, cyano ou acyle en $C_1$ à $C_3$ ;

$R_5$ et $R_6$, qui peuvent être identiques ou différents, désignent un atome d'hydrogène, un groupe méthyle, hydroxy, carboxy (alcoxy en $C_1$ à $C_4$) carbonyle, hydroxyméthyle, phényle ou p-chlorophényle ;

$R_7$ et $R_8$ désignent un atome d'hydrogène ou un groupe méthyle ;

j et k sont des indices de 0 à 3, mais dont le total n'est pas supérieur à 4 ;

m et n sont des indices de 0 à 3, mais dont le total n'est pas supérieur à 4 ;

A désigne $—CH_2—$ ou $—CH_2—CH_2—$ : ou

$R_5$ et $R_7$ ensemble ou $R_6$ et $R_8$ ensemble forment un groupe oxo, sous réserve que k ou m soit différent de 0 ; ou

$R_5$ et $R_7$ ensemble ou $R_6$ et $R_8$ ensemble forment un groupe oxo, sous réserve que k et m soient différents de 0 ;

$R_9$ et $R_{10}$, qui peuvent être identiques ou différents, désignent un atome d'hydrogène ou un à quatre substituants méthyle sur l'atome de carbone d'un cycle pipérazine ($A = —CH_2—$) ;

$R_{11}$, $R_{12}$, $R_{13}$ et $R_{14}$, qui peuvent être identiques ou différents, désignent un atome d'hydrogène ou un groupe méthyle ou

$R_{11}$ et $R_{12}$ et/ou $R_{13}$ et $R_{14}$ forment ensemble un groupe oxo ; et

X désigne une chaîne alkylène en $C_1$ à $C_2$, qui est substituée le cas échéant par un groupe hydroxy, ou un de ses sels d'addition aux acides physiologiquement inoffensifs, en association avec des adjuvants et/ou des supports habituels.

11. Préparations pharmaceutiques contenant comme principe actif un composé suivant la revendication 10 en association avec d'autres substances pharmacodynamiquement actives ainsi que des adjuvants et/ou supports habituels.

12. Procédé de confection de préparations pharmaceutiques suivant la revendication 10, caractérisé en ce qu'on transforme un composé suivant la revendication 10, avec des adjuvants ou supports galéniques habituels, en formes d'utilisation pharmaceutiques habituelles.

13. Procédé de confection de préparations pharmaceutiques suivant la revendication 11, caractérisé en ce qu'on transforme un composé suivant la revendication 10, en association avec d'autres substances pharmacodynamiquement actives, ainsi que des adjuvants et/ou des supports habituels, en des formes d'utilisation pharmaceutiques habituelles.

14. Utilisation d'un composé suivant la revendication 10 pour la confection de préparations pharmaceutiques pour le traitement des réactions allergiques et d'inflammations chez les animaux à sang chaud.